# EUROPEAN PATENT SPECIFICATION

(11) **EP 1 797 194 B1**
(45) Date of publication and mention of the grant of the patent: **02.12.2009**
(21) Application number: 05803922.3
(22) Date of filing: 05.10.2005
(51) Int. Cl.: C12Q 1/00

(54) **STABLE THREE ENZYME CREATININE BIOSENSOR**
STABILER KREATININ-BIOSENSOR MIT DREI ENZYMEN
BIOCAPTEUR DE CREATININE POUR TROIS ENZYMES STABLE

(30) Priority: 05.10.2004 US 616149 P
(43) Date of publication of application: 20.06.2007
(73) Proprietor: Siemens Healthcare Diagnostics Inc., Tarrytown, NY 10591 (US)
(72) Inventor: BERBERICH, Jason, Pittsburg, PA 15218 (US); BODEN, Mark, W., Harrisville, RI 02830 (US); CHAN, Andy, D., C., Franklin, MA 02038 (US); RUSSELL, Alan, Gibsonia, PA 15044 (US)
(74) Representative: Maier, Daniel Oliver
(86) International application number: PCT/US2005/035959
(87) International publication number: WO 2006/042001

(56) References cited:
- DE-A1-102004 003 793
- US-B1- 6 241 863
- TSUCHIDA T ET AL: "MULTI-ENZYME MEMBRANE ELECTRODES FOR DETERMINATION OF CREATININE AND CREATINE IN SERUM" QUALITY CONTROL CLIN. CHEM. TRANSACTIONS INTERNATIONAL SYMPOSIUM, vol. 29, no. 1, 1983, pages 51-55, XP001008686
- KUBO & I KARUBE I: "Immobilization of creatinine deiminase on a substituted poly(methylglutamate) membrane and its use in a creatitine sensor" ANALYTICA CHIMICA ACTA, ELSEVIER, AMSTERDAM, NL, vol. 187, 1986, pages 31-37, XP002081413 ISSN: 0003-2670
- YAMATO, H. ET AL: "A polypyrrole/three enzyme electrode for creatinine detection" ANAL. CHEM., vol. 67, no. 17, 1 September 1995 (1995-09-01), pages 2776-2780, XP002379867
- ERLENKÖTTER ANSGAR ET AL: "Biosensors and flow-through system for the determination of creatinine in hemodialysate." ANALYTICAL AND BIOANALYTICAL CHEMISTRY. JAN 2002, vol. 372, no. 2, January 2002 (2002-01), pages 284-292, XP002379868 ISSN: 1618-2642
- BERBERICH J A ET AL: "A stable three-enzyme creatinine biosensor. 1. Impact of structure, function and environment on PEGylated and immobilized sarcosine oxidase" ACTA BIOMATERIALIA, ELSEVIER, AMSTERDAM, NL, vol. 1, no. 2, March 2005 (2005-03), pages 173-181, XP004748995 ISSN: 1742-7061
- BERBERICH J A ET AL: "A stable three enzyme creatinine biosensor. 2. Analysis of the impact of silver ions on creatine amidinohydrolase" ACTA BIOMATERIALIA, ELSEVIER, AMSTERDAM, NL, vol. 1, no. 2, March 2005 (2005-03), pages 183-191, XP004748996 ISSN: 1742-7061
- BERBERICH J A ET AL: "A stable three-enzyme creatinine biosensor. 3. Immobilization of creatinine amidohydrolase and sensor development" ACTA BIOMATERIALIA, ELSEVIER, AMSTERDAM, NL, vol. 1, no. 2, March 2005 (2005-03), pages 193-199, XP004748997 ISSN: 1742-7061

## Description

### FIELD OF THE INVENTION

The present invention relates generally to the field of diagnostic medicine and, more specifically, to methods for producing a multiple-use biosensor for amperometric creatinine determination that includes a biopolymer of immobilized enzymes.

### BACKGROUND OF THE INVENTION

The determination of creatinine levels in biological fluids is an increasingly important clinical necessity. Amperometric biosensors have been developed based on a three-enzyme system which converts creatinine to amperometrically measurable hydrogen peroxide. Due to the complexity of the three-enzyme system, development of these biosensors has been slow.

Incorporation of enzymes into polymer networks through multi-point attachment is a rapid and effective general strategy for enhancing the stability of enzymes, while retaining activity. This strategy involves the production of bioplastics in a single step, employing oligomers capable of chemical reaction with specific functionalities on the enzyme surface..

The utility of enzymes in biosensors is limited by their stability. Clinical blood analyzers require enzymes to be used over and over while in contact with whole blood. Many blood analyzers operate at 37 °C which further limits enzyme stability. A variety of immobilization procedures are described in the literature for use with biosensors. Although most of the procedures described suggest utility for biosensors, they are often not tested under conditions that would be applicable under real-life conditions, such as room temperature or at 37 °C while in contact with fluid. Enzyme immobilization is especially critical for continuous use biosensors where enzyme leaching can be a concern.

Enzyme immobilization into a "biopolymer" by multipoint covalent attachment affords a straightforward and convenient method for preparation of immobilized enzymes for biosensors. Not only does multipoint covalent immobilization prevent enzyme leaching, but it also increases enzyme stability to heat, pH, organic solvents, peroxides and proteolytic and microbial degradation.

Thus, there is a need for the development of multiple-use biosensors that have incorporated as a component stable enzymes that retain activity. The present invention meets this need and provides related advantages.

### SUMMARY OF THE INVENTION

The invention provides methods for preparing a stable, multiple-use three enzyme biosensor for the amperometric determination of creatinine in biological liquids that has a useful lifetime significantly beyond that of presently available amperometric biosensors. The biosensor prepared by the methods of the invention encompasses a plurality of immobilized enzymes that are applied to the biosensor as an enzyme-polymer composition. The enzymes, which can include creatinine amidohydrolase, creatine amidinohydrolase and sarcosine oxidase, are immobilized into the enzyme-polymer composition simultaneously as well as applied to the biosensor simultaneously. Prior to being immobilized, the enzymes are chemically modified by attaching one or more polyethylene glycol (PEG) chains per enzyme monomer. The polymer component can be provided by a polyurethane membrane. The invention also provides a method of preparing a biosensor that limits the diffusion of silver ions emanating the reference electrode, thereby preventing contact between the silver ions and the enzymes. Related methods of preparing an enzyme-polymer composition for incorporation into a multiple-use three enzyme biosensor for the amperometric determination of creatinine in biological liquids also are provided. The invention also provides multiple-use biosensors and enzyme-polymer compositions prepared by the methods disclosed.

### BRIEF DESCRIPTION OF THE DRAWINGS

Figure 1 shows activity retention of PEGylated MSOX modified using PEG-NCO at pH 7.5 (closed circles) and pH 8.5 (closed squares).
Figure 2 shows activity retention of MSOX after PEGylation in the presence of inhibitors (10: 1 (solid) and 10: 1 (white) isocyanate to amine ratio). The inhibitor concentration was 50 mM.
Figure 3 shows stability of sarcosine oxidase as a function of number of PEGs attached at 37°C. Native enzyme (open squares); one PEG attached (closed squares); two PEGs attached (closed circles).
Figure 4 shows relative activity of MSOX containing polyurethanes as a function of enzyme content.
Figure 5 shows reusability of MSOX containing polyurethane hydrogels.
Figure 6 shows leaching of sarcosine oxidase from polyurethane hydrogels. 1 mg MSOX/g polymer (closed triangles); 2 mg MSOX/g polymer (open squares).
Figure 7 shows stability of MSOX containing polyurethane hydrogels stored in buffer at 37°C. Rates were normalized to the rate of oxygen consumption after the first day. Native enzyme (closed circles); immobilized enzyme (open circles).
Figure 8 shows ion induced irreversible inhibition of sarcosine oxidase activity. Incubation time: 5 min (closed circles); 1 her (open squares); 3 hr (closed triangles); 5 hr (open circles); 21 hr closed squares).
Figure 9 shows stability of creatine amidinohydrolase as a function of number of PEGs attached at 37°C. Native enzyme (closed diamonds); one PEG attached (closed triangles); three PEGs attached (closed circles); five PEGs attached (closed squares).
Figure 10 shows stability of creatine amidinohydrolase-containing polyurethane hydrogels stored in buffer at 37°C. Rates were normalized to the rate of oxygen consumption after the first day. Native enzyme (open circles); Immobilized enzyme: 10 units/g polymer (closed triangles); 50 units/g polymer (closed squares); 100 units/g polymer (closed circles).
Figure 11 shows silver-induced deactivation of creatine amidinohydrolase in solution. Incubation time: 5 min (closed circles); 15 min (closed squares).
Figure 12 shows protection of creatine amidinohydrolase from silver-induced inactivation.
Figure 13 shows residue fluctuations in creatinase. Panel **(A)** Comparison of predicted (by GNM; shown in blue) and experimental (X-ray crystallographic; red) B-factors. Panel **(B)** Color-coded ribbon diagram of the monomer showing the most flexible regions (peaks in panel a) in red, and the least flexible (minima) in blue. Panel **(C)** Ribbon diagram illustrating the relative positions of the monomers A and B with respect to the ligand.
Figure 14 shows distribution of mobilities in the dominant global mode of creatinease dimer. The catalytic residues Phe62, Arg64, His231, Tyr257, Glu261, Arg 334 and Glu357 are shown by the blue open circles and Cys60, Cys249 and Cys297 are shown by orange squares, in both monomers (separated by the dashed line). Red arrows indicate the catalytic residues that coordinate the CMS in the examined crystal structure.

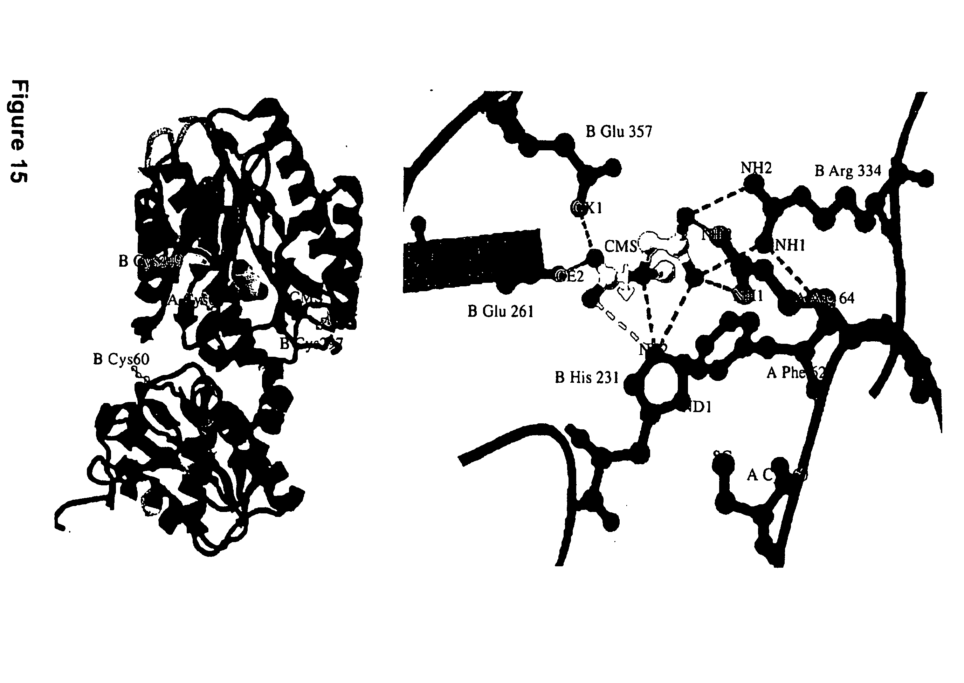

Figure 15 in Panel **(A)** shows color-coded ribbon diagram illustrating the mobility of creatinase residues in global motions. The colors blue- green-yellow-orange-red are used in the order of increasing mobility. Creatine analog, CMS, is shown in space-filling representation. The yellow arrows indicate the three cysteines (shown in ball-and-stick) on monomer B, and the white arrow the Cys60 on monomer A. Atoms in CMS and cysteines are colored by CPK convention. Panel **(B)** shows dynamics near the active site. Creatine analog, CMS, and key catalytic residues are shown in ball-and-stick. Catalytic residues' sidechains and their associated backbone (ribbon) are colored according to their global mobilities (same as panel a, shown from a different perspective for clarity). Hydrogen bonds are shown in dashed lines. The location of the peptide bond cleavage during catalytic electron transfer is shown by the white arrow. The moderate mobility of Glu261 and Glu357, which form hydrogen bonds with the nitrogen atom in the guanidine group of CMS can facilitate the peptide bond cleavage. Phe62 and Arg64 join the catalytic pocket from the other chain.

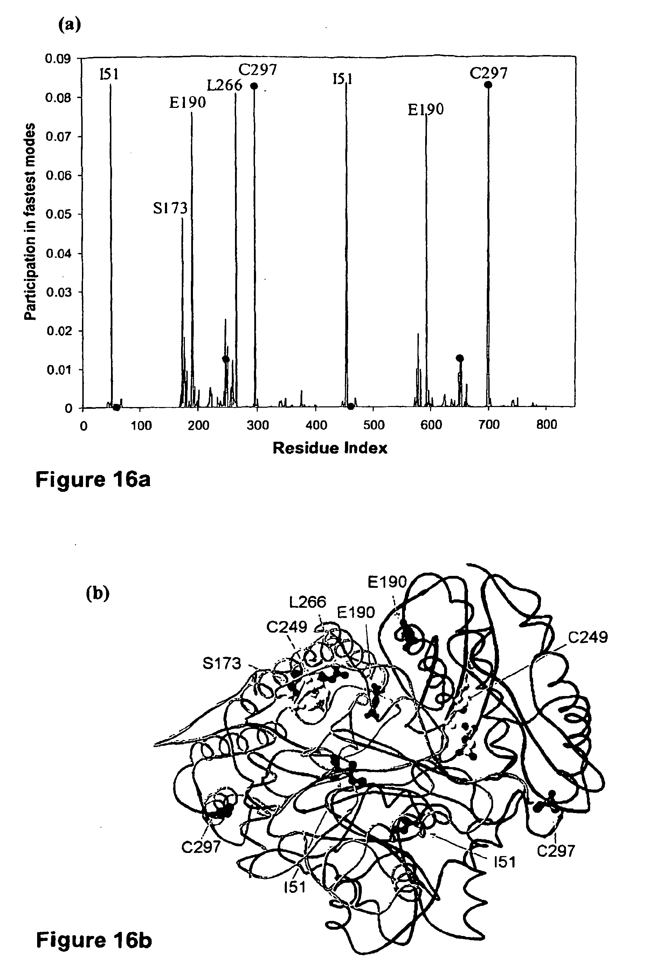

Figure 16 in Panel **(A)** shows fluctuations in the high frequency modes. Peaks (labeled by the residue types and numbers) reveal the centers of localization of energy subject to the highest frequency vibrations. Cys60, Cys249 and Cys297 are shown by red circles. The highest peak at Cys297 indicates that this residue significantly contributes to folding/stability. Panel **(B)** shows the same results mapped onto the crystal structure of creatinase. The α-carbon trace of monomer B is shown in black, and that of monomer A in gray. The sidechains of peak residues in panel (a) are displayed in ball-and-stick, colored red (highest peaks) or yellow (moderate peaks). CMS is colored by CPK conventions. We note that S 173, C249 and L266 in monomer A form close contacts in space, indicative of a folding nucleus.
Figure 17 shows activity retention of PEGylated creatinine amidohydrolase as a function of average number PEG chains covalently attached.
Figure 18 shows stability of creatinine amidohydrolase as a function of number of PEGs attached at 37 °C. Native enzyme (closed circles); one PEG attached (closed squares); two PEGs attached (closed triangles); three PEGs attached (open circles); four PEGs attached (open triangles); five PEGs attached (open squares).
Figure 19 shows relative activity of creatinine amidohydrolase containing polyurethanes as a function of enzyme content.
Figure 20 shows reusability of creatinine amidohydrolase containing polyurethane gels.
Figure 21 shows stability of creatinine amidohydrolase-containing polyurethane hydrogels stored in buffer at 37 °C. Rates were normalized to the rate of oxygen consumption after the first day. Native enzyme (closed circles); immobilized enzyme (open circles).
Figure 22 shows silver ion induced deactivation of creatinine amidohydrolase in solution. Incubation time: 5 min (open circles); 1 hr (closed squares).
Figure 23 shows stability of 3-enzyme containing polyurethane membranes stored in 50 mM phosphate buffer (pH 7.5) at 37 °C with (closed squares) and without (closed circles) planar sensors.
Figure 24 shows activity retention of 3-enzyme electrodes stored in buffer at 37 °C. Electrodes were prepared with (open squares) and without (closed circles) cellulose acetate cover membranes.

### DETAILED DESCRIPTION OF THE INVENTION

The invention provides methods for preparing a stable, multiple-use three enzyme biosensor for the amperometric determination of creatinine in biological liquids that has a useful lifetime that extends significantly beyond that of presently available amperometric biosensors.

As disclosed herein, the enzymes creatinine amidohydrolase, creatine amidinohydrolase and sarcosine oxidase can be effectively modified and incorporated into enzyme-polymer composotions. The immobilization of the enzymes into enzyme-polymer compositions according to the methods of the invention significantly improves the enzyme stability and increases the half-life. The enzyme-polymer compositions and biosensors prepared according to the methods of the invention have considerable enzyme stability and allow for multiple-use of the biosensor extending for more than 4 days, more than 6 days, more than 8 days, more than 10 days, more than 12 days, more than 15 days, more than 20 days, more than 25 days, more than 30 days.

The biosensor prepared by the methods of the invention encompasses a plurality of immobilized enzymes that are applied to the biosensor as an enzyme-polymer composition. The enzymes, which can include creatinine amidohydrolase, creatine amidinohydrolase and sarcosine oxidase, can be immobilized into the enzyme-polymer composition simultaneously as well as applied to the biosensor simultaneously. In other embodiments, enzymes can be immobilized and added in a step-wise manner or any combination of desired by the user.

A critical step in the development of biosensors is effective enzyme immobilization. The invention is based, in part, on the discovery that it is possible to successfully immobilize the three enzymes used in amperometric creatinine biosensors, creatinine amidohydrolase creatine amidinohydrolase, and sarcosine oxidase, into membranes using polyurethane prepolymers while retaining sufficient enzymatic activity. As exemplified herein, the three enzymes can be covalently modified and incorporated into polyurethane hydrogels where they exhibit improved stability when compared to the native enzymes in solution at 37°C. The biosensor prepared according to the methods of the invention can be used at a temperature desired by the user and depending on the particular application, for example, at room temperature as well as at 37°C.

In a preferred embodiment, the invention provides a method for preparing a multiple-use three enzyme biosensor for the amperometric determination of creatinine in biological liquids, the biosensor containing a plurality of immobilized enzymes, the method comprising applying to the biosensor an enzyme-polymer composition comprising the plurality of immobilized enzymes.

The three-enzyme system converts creatinine to amerometrically measurable hydrogen peroxide H₂O₂.

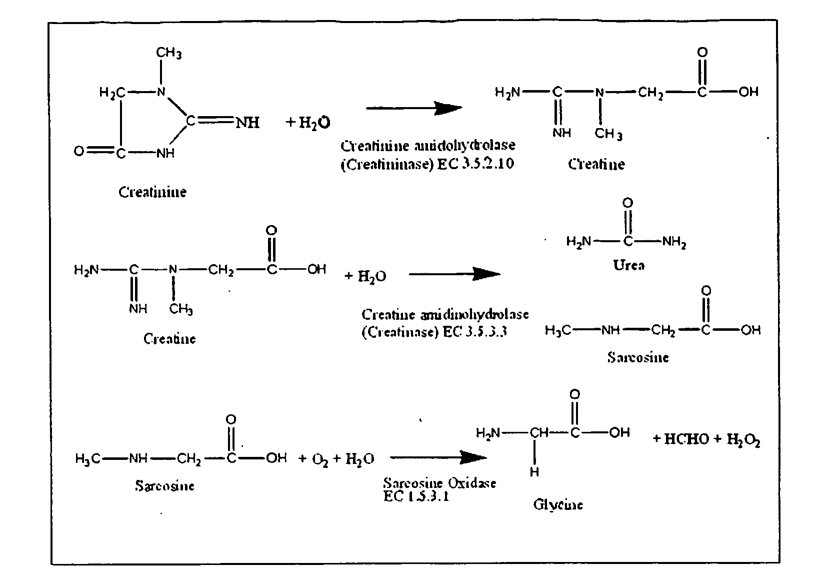

It is contemplated that all three enzymes that are components of this system are immobilized in the enzyme-polymer composition. However, embodiments in which only one or two of the three enzymes are immobilized into the enzyme-polymer composition also represent useful embodiments of the invention. Thus, at least one or at least two or all three of the enzymes creatinine amidohydrolase, creatine amidinohydrolase and sarcosine oxidase can be immobilized into the enzyme-polymer composition that is applied to a biosensor of the invention. The enzymes can be immobilized into the enzyme-polymer composition simultaneously in a single step. Consequently, the enzymes can be applied to the biosensor simultaneously as part of a polymer sheet or membrane, for example, a polyurethane membrane.

The methods of the invention for preparing a stable, multiple-use three enzyme biosensor for the amperometric determination of creatinine in biological liquids further includes the initial step of chemically modifying the enzymes by attaching one or more polyethylene glycol (PEG) chains per enzyme monomer. The conjugation of enzyme monomers with water soluble polymeric modifiers, especially, the conjugation with polyethylene glycol (PEG), often called "PEGylation" is contemplated as an initial step. Bioconjugation of the enzyme monomers with water-soluble polymeric modifiers increases their molecular size and steric hindrance and improve the half-lives of the enzymes.

A biosensor according to the invention has at least one working, at least one reference and at least one counter electrode and has applied to its surface one, two or three of the enzymes, which have been immobilized as disclosed by the method of the invention. As disclosed herein, the enzymes can become part of the biosensor by being incorporated into an enzyme-polymer composition, for example, a polyurethane membrane, that is applied to the sensor.

If desired by the user, the sensor can have, for example, two working electrodes, each containing at least one, at least two or three immobilized enzymes. For example, one electrode may contain two of the three enzymes, while a second working electrode contains all three. Any combination and permutation of enzymes and electrodes can be selected by the used based on the desired embodiment of the invention that is to be performed. In a further embodiment, the biosensor is made up of two three-electrodes systems, the first electrode system comprising the enzymes creatinine amidohydrolase, creatine amidinohydrolase and sarcosine oxidase and serving for the determination of the sum of creatinine and creatine and the second electrode system comprising the enzymes creatine amidinohydrolase and sarcosine oxidase and serving for the determination of creatine, whereby the result of the second electrode system is deducted from the result of the first one for the determination of creatinine. If desired, the biosensor comprises a further electrode system serving for the elimination of electrochemical interferences.

Enzymes can be susceptible to inhibition by silver, which can cause inactivation even if present in micromolar quantities. Susceptibility to silver ions can be important when using the enzymes in amperometric creatinine biosensors with silver-containing electrodes. As described below, a three-enzyme-polymer applied to silver electrode bodies can cause deactivation by silver ions. For example, creatine amidinohydrolase is highly susceptible to inhibition by silver. Addition of silver scavenging thiol-containing molecules can be effective in preventing the loss of enzyme activity due to silver. Based on GNM analysis of the molecular dynamics of creatine amidinohydrolase, two of the cysteine residues, Cys60 and Cys297 in both chains were identified as important in controlling the functional dynamics of the enzyme and play a role in destabilizing the enzyme and leading to inactivation. Significantly, Cys297 is surrounded by negatively charged residues that attract positively charged silver near this residue. The use of creatine amidinohydrolase in a biosensor therefore requires protection from silver ions, which can be accomplished by utilizing a mimetic of the enzyme that has decreased silver susceptibility, adding silver scavengers or, in preferred embodiments, covering the electrode with a material that prevents contact between silver ions and the enzyme.

In view of the above it is contemplated that, in embodiments where the reference electrode is an Ag/AgCl (silver/chloride) electrode, the reference electrode can be covered with a material that limits diffusion of silver ions emanating from said reference electrode, thereby preventing contact between the silver ions and the enzymes. It is contemplated that the use of a cellulose acetate cover membrane to separate the electrode from the enzyme-polymer composition, for example, the polyurethance membrane, can slow the leaching of silver from the electrodes can improve sensor half-life. Furthermore, improvements in sensor design to prevent the leaching of silver ions and reduce enzyme contact with the sensor can be used to increase the use-life of creatinine biosensors and increase their utility in clinical whole blood analysis. Generally, an electrode in a biosensor according to the invention can consist of carbon, metal, metal oxides or a mixture of carbon and metal or metal oxides. Furthermore, it is contemplated that the electrodes are applied on a nonconducting substrate.

In a preferred embodiment, the biosensor is made up of two three-electrodes systems, the first electrode system comprising the enzymes creatininase, creatinase and sarcosine oxidase and serving for the determination of the sum of creatinine and creatine and the second electrode system comprising the enzymes creatinase and sarcosine oxidase and serving for the determination of creatine, whereby the result of the second electrode system is deducted from the result of the first one for the determination of creatinine. If desired, the biosensor can contain a further electrode for the elimination of electrochemical interferences.

The methods of the invention encompass the chemical modification and immobilization of sarcosine oxidase into an enzyme-polymer composition, also referred to as a biolpolymer, for example into a polyurethane polymer. While the invention method is exemplified with polyurethance, the skilled person will appreciate that any polymer that allows for incorporation of the enzymes into the polymer network in a manner that enhances the stability of the enzymes, while retaining activity, is contemplated as useful for practicing the methods of the invention, for example, polyurethane, cyanate polymers, polyvinyl chloride, polyester, polycarbonate, vinyl acetate copolymer, nylon, poly (1,4-butleneterephthalate), cellulose propionate, ethylene/acrylic acid copolymer, polybutadiene, polyethylene, polypropylene, polyimide, acrylic film, polystyrene, and polyvinyl fluoride.

In a preferred embodiment, the methods provided herein encompass the production of an enzyme-polymer composition is performed in a single step, employing oligomers capable of chemical reaction with specific functionalities on the enzyme surface. The enzymes are attached to the polymer in a manner that provides protein retention, for example, by means of crosslinking, covalent binding or matrix inclusion, so that the advantages of immobilization can be maximized.

The invention is based, in part, on the discovery of the particular structure-function-environment relationship required to maintain sarcosine oxidase in a polymeric sensor environment. Sarcosine oxidase (EC 1.5.3. 1) catalyzes the oxidative demethylation of sarcosine (N-methylglycine) and forms equimolar amounts of formaldehyde, glycine, and hydrogen peroxide. Sarcosine oxidase from the *Arthrobacter sp.* is a monomer with a molecular weight of 43 kDa (Nishiya and Imanaka, J Ferm Bioeng 75:139-244 (1993)). The monomeric sarcosine oxidases (MSOX) are flavine proteins that contain mole of flavine adenine dinucleotide (FAD) that is covalently linked to the enzyme by a cysteine residue (Trickey et al., Structure 7:331-345 (1999)). In certain embodiments the enzyme sarcosine oxidase is contacted with an inhibitor, for example, pyrrole-2-carboxylic acid or (methylthio)acetic acid, in an amount effective to prevent inactivation during the chemical modification step. As described herein, sarcosine oxidase was inactivated when modified by a single molecule of PEG-NCO and it was discovered that two irreversible inhibitors, (methylthio)acetic acid and pyrrole-2-carboxylic acid, were effective in preventing enzyme inactivation during modification allowing the enzyme to be successfully and irreversibly immobilized during polymerization by chemical crosslinking into polyurethane hydrogel-forming polymers, retaining activity and stability sufficient for use of approximately 30 days in a buffered solution at 37°C.

Creatine amidinohydrolase (creatinase, EC 3.5.3.3) is a homodimer with subunit molecular weights of approximately 45 kDa. The two active sites of the protein are at the interface of the monomers being shared by each monomer and only the dimer is active. While the enzyme has a low functional stability, additives such as reducing agents, proteins and polyols have been shown to increase the stabilty of the enzyme (Schumann et al., Biol Chem 374:427-434 (1993a)). The low intrinsic stability of creatinase has prompted the use of protein engineering to improve stability (Schumann, et al., Protein Science 2:1612-1620 (1993b)). As disclosed herein, creatine amidinohydrolase can be effectively modified by PEG-NCO and covalently incorporated into polyurethane materials via modification by isocyanate prepolymers. Creatine amidinohydrolase was successfully and irreversibly immobilized by chemical crosslinking into polyurethane polymers and retained significant activity and sufficient stability to be used for 30 days in a buffered solution at 37°C.

The following examples are intended to illustrate, but not to limit the invention.

### EXAMPLE I

### MODIFICATION, IMMOBILIZATION AND-MAINTENANCE OF SARCOSINE OXIDASE IN A POLYMERIC SENSOR ENVIRONMENT

This example describes the immobilization of sarcosine oxidase in polyurethane polymers using PEG-NCO.

### A. Materials and Protocols

Sarcosine oxidase (from *Arthrobacter sp.,* SAO-341) was purchased from Toyobo Co., Ltd. Horseradish peroxidase was purchase from Sigma-Aldrich (St. Louis, MO). All enzymes were used without further purification. PEG-NCO (Mw 5 000) and PEGSPA (M_{w} 5,000) were obtained from Shearwater Polymers Inc. (Huntsville, AL). Hypol 2060G prepolymer was purchased from Hampshire Chemical (Lexington, MA). All other reagents were purchased from Sigma-Aldrich Chemicals (St. Louis, MO) and were of the highest purity available.

### PEGylation of sarcosine oxidase

Sarcosine oxidase was dissolved in an aqueous buffer (50 mM phosphate buffer, pH 7.5 or 50 mM borate buffer, pH 8.5) at a concentration of 1 mg/mL. PEG-NCO or PEG-SPA was added in excess to the enzyme at a molar ratio of 1:100 to ensure complete enzyme modification. In some experiments sarcosine oxidase inhibitors (50 mM methylthioacetic acid or 50 mM pyrrole-2-caroxylic acid) were added to help prevent inactivation of the enzyme. The reaction mixture was mixed for 30 min followed by dialysis (12 000 M_{w} cutoff) against 50 mM phosphate buffer (Drevon et al., Biomacromolecules 2:764-771 (2001)).

### Synthesis of sarcosine oxidase-containing polyurethane

Hypol prepolymer 2060G (0.4 g), a toluene diisocyanate based prepolymer, was added to a buffered solution (3.6 g of 50 mM phosphate buffer, 50 mM inhibitor, pH 7.5 containing sarcosine oxidase (0 to 200 units enzyme per gram of prepolymer). The aqueous polymer solution was vigorously mixed for 30 s in a weigh boat until the onset of gelation. Polymerization was very rapid and gelation usually took place within one minute.

### Characterization of enzyme modification

MALDI-MS analyses were performed using a Perspective Biosystems Voyager Elite MALDI- TOF. The acceleration voltage was set to 20 kV in a linear mode. I µL of PEGylated enzyme solution (0.1 mg/mL) was mixed with 1 ÿL of matrix solution (0.5 mL water, 0.5 mL of acetonitrile, 1 µL of trifluoracetic acid, and 10 mg of sinapinic acid) and then spotted on the target plate. Spectra were recorded after evaporation of the solvent mixture and were calibrated externally with equine cytochrome C (12,361.96 Da (ave)), rabbit muscle aldolase (39,212.28 Da (ave)) and bovine serum albumin (66,430.09 Da (ave)).

### Measurement of sarcosine oxidase activity using an end point assay

The production of hydrogen peroxide was measured by using the 4-aminoantipyrene-peroxidase system (Nishiya and Imanaka, Appl. Environ. Microbiol. 62:2405-2410 (1996)). Typically, an enzyme solution (0.05 mL) was incubated with a mixture (1.0 mL total) of 95 mM sarcosine, 0.47 mM 4-aminoantipyrene, 2. mM phenol, 0.045% Triton X- 100, 50 mM sodium phosphate (pH 8.0) and 5 units/mL of horseradish peroxidase at 37°C for 10 minutes. The reaction was terminated by addition of 2.0 mL of 0.25% SDS solution and the absorbance at 500 nm was measured. One unit was defined as the amount of enzyme that catalyzed the oxidation of 1).mol of substrate per minute.

### Measurement of sarcosine oxidase activity usine an oxygen monitor assay

The initial rate of oxygen consumption was also measured at 37 °C with a Clark oxygen electrode from Yellow Springs Instruments (Yellow Springs, OH). The reaction was initiated by adding an enzyme solution (1 µL) or an enzyme-containing polymer (10-100 mg cut into small pieces) to 5.0 mL of substrate (50 mM sarcosine in 50 mM phosphate buffer, pH 7. 5). Before measurement, the assay solution was allowed to equilibrate to 37 °C with air. Oxygen consumption was measured for 5 to 10 minutes.

### Thermostability of native sarcosine oxidase

Sarcosine oxidase was added to a buffered medium (50 mM sodium phosphate, 2 mM EDTA, pH 7.5). The native enzyme concentration used was 0.06 mg/ml. The activity of sarcosine oxidase was followed over time at room temperature (22 °C), and at 4 °C and 37 °C using the end point assay described above.

### Thermostability of PEG-modified sarcosine oxidase

Thermoinactivation of PEG-sarcosine oxidase was monitored at 37 °C in buffer (50 mM sodium phosphate, 2 mM EDTA, pH 7. 5) as described for the native enzyme. The enzyme concentration in all samples was adjusted to 0.05 mg/ml.

### Thermostability of immobilized sarcosine oxidase

Enzyme polymer samples were cut into small pieces and added to buffer (50 mM phosphate, pH 7.5) and incubated at 37 °C. Samples were removed over time and assayed for enzymatic activity using the oxygen electrode.

### Silver inhibition of sarcosine oxidase

To determine the effect of silver ions on sarcosine oxidase, 0.07 mg/mL sarcosine oxidase were incubated in 20 mM Tris-HCl (pH 7.5) with silver nitrate (0 to 1 mM) at room temperature. Samples were removed periodically and assayed using the end point assay for sarcosine oxidase activity.

### B. Results

### Effect of PEGylation on enzyme activity and stability

Since immobilization of the enzyme into polyurethane polymers involves chemical modification of the enzyme by reactions involving isocyanate with nucleophilic residues on the enzyme surface it is convenient to model the process of chemical modification using soluble polymers. Modification using this technique allows us to mimic the effect of covalent immobilization on enzyme activity while still working with a soluble enzyme. Once the enzyme is incorporated into a polymer, mass transfer effects can complicate the analysis.

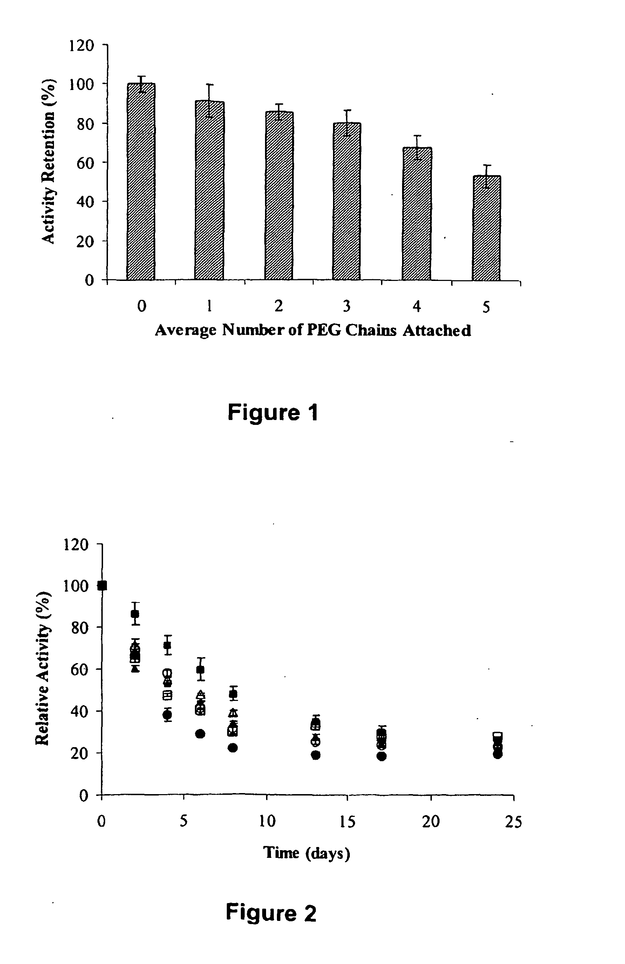

Monomeric sarcosine oxidase was modified using PEG-NCO (5,000 MW) at pH 7.5 (50 mM phosphate buffer) and pH 8.5 (50 mM borate buffer) using different ratios (NCO/enzyme). The amount of native enzyme remaining was quantified using MALDI-TOF analysis and plotted: versus the activity retention of sarcosine oxidase following modification (Figure 1). When the enzyme was modified at pH 7.5, the percentage of activity retained was proportional to the percentage of unmodified enzyme. This indicates that a critical residue from the perspective of the enzyme mechanism (or group of residues) dominates the reaction with the isocyantate. However, modification at pH 8.5 led to increased activity retention probably due to the increased nucleophilicity of the ÿ-amines of lysine (average pKa = 9.3 - 9.5) with increased pH.

Since a single modification by isocyanate appeared to deactivate sarcosine oxidase, a method will be required to protect the enzyme during immobilization within polyurethane polymers. If modification is occurring at or near an active site residue, an inhibitor can be added to solution to help increase activity retention by blocking the active site cleft. Although this method of protection is often discussed, the literature contains precious few examples of where inhibitor protection strategies work effectively. A number of inhibitors have been identified for sarcosine oxidase (Wagner et al., Biochem 39:8813-8824 (2000)). Pyrrole-2-carboxylic acid (Ki=1.37 mM) and (methylthio)acetic acid (Ki =2.60 mM) were selected since they had low Ki values and do not contain functional groups that are highly reactive with isocyanate. The native substrate, sarcosine (Km = 0.6), was not used as a protecting agent since the catalytic oxidation product, hydrogen peroxide, can inactivate the enzyme and can also oxidize the PEG backbone. Modification of sarcosine oxidase at pH 7. 5 with either inhibitor showed considerably improved activity retention compared to modification without inhibitor (Figure 2). MALDI- TOF analysis confirmed that the majority of the enzyme was modified with PEG and that the presence of the inhibitor had minimal effect on the modification process itself.

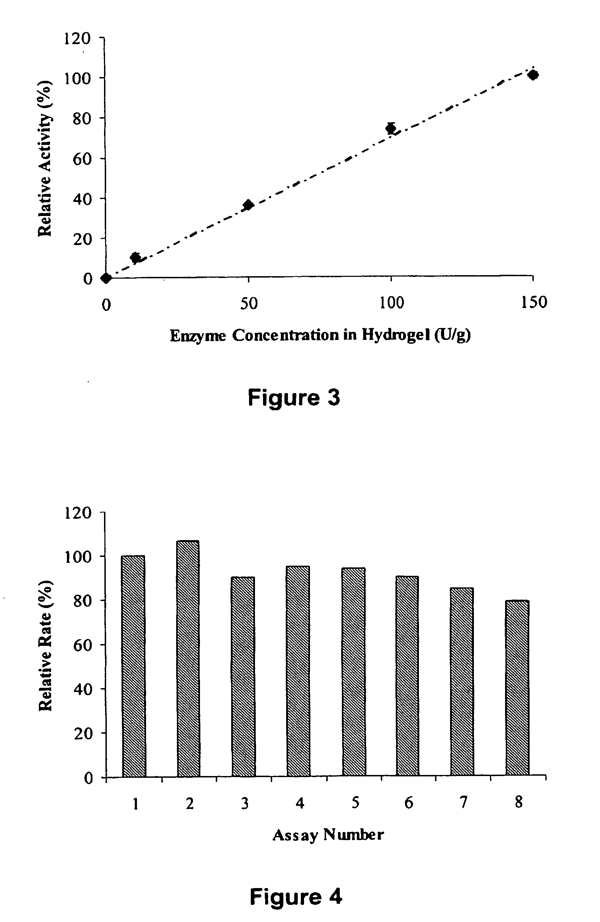

Stability of native and PEG-modified sarcosine oxidases were measured at 37 °C in 50 mM phosphate buffer (Figure 3). Enzyme with an average of one PEG chain attached per molecule of enzyme had a stability similar to native enzyme (half-life of 7 days). Enzyme with an average of three PEG chains attached per molecule of enzyme had an improved half-life (17 days).

### Discovery of the nature of chemical modification

Isocyanates are capable of reacting with amino, sulfhydryl, carboxyl, phenolic hydroxyl, imidazole, and phosphate groups in proteins (Means and Feeney, Chemical modification of proteins, San Francisco:Holden-Day, Inc. (1971)); however, only the reaction with amino groups results in the formation of a stable product. Reactions with sulfhydryl, imidazole, tyrosyl, and carboxyl groups give relatively unstable adducts that can decompose upon dilution or change in pH (REF).

Inactivation by cyanate and isocyanate has been reported for a number of enzymes: pepsin (Rimon and Perlmann, J Biol Chem 243: 3566-3572 (1968), papain (Sluyterman, Biochim Biophys Acta 139:439-449 (1967)), trypsin and chymotrypsin (Shaw et al., J Biol Chem 239 :PC671-673 (1964); Brown and Wold, Biochemistry 12:835-40 (1973)), and glutathione reductase (Jochheim and Bailie, Biochem Pharmacol 47:1197-1206 (1994)). The proteolytic activity of pepsin was inhibited when tyrosine residues were carbamylated by potassium cyanate; however treatment with hydroxylamine was effective in reversing the inactivation by decarbamylating the residues (Rimon and Perlman, supra, 1968). Papain is also inactivated by cyanate; in fact, the active site thiol of papain is about 3000 times more reactive with cyanate than the thiol group of free cysteine (Sluyterman, supra, 1967). The inactivation is reversible upon dilution due to the lability of the carbamylated sulfhydryl group. Chymotrypsin was also shown to be inactivated by cyanate (Shaw et al., supra, 1964). Modification of the active site serine is the cause of the inactivation and similar effects have been reported for trypsin and subtilisin (Shaw et al., supra, 1964).

In order to determine if sarcosine oxidase modified with isocyanate was modified at a residue giving an unstable adduct (a modified sulfhydryl or tyrosyl residue for example), the enzyme was treated in a number of ways to attempt to reactivate the enzyme activity. Neither dilution, nor dialysis overnight at pH 7.5 was successful in reactivating the enzyme. Treatment with hydroxylamine is a method frequently used to remove weakly bound conjugates from enzymes (Smyth, J. Biol. Chem. 242:1592-1:598 (1967)). Treatment of isocyanate-modified sarcosine oxidase with hydroxylamine (500 mM, pH 7) for up to 24 hrs was ineffective in reviving the enzyme. Thus, the PEG-isocyantate has reacted with an amine on the surface of the protein and thereby inactivated the enzyme.

In order to determine if inactivation was specific for isocyanate, or whether other amine-modifying PEGs would have a similar effect, sarcosine oxidase was modified using PEG-SPA. PEG-SPA is an NHS-modified PEG that forms stable amide linkages with amines (Hermanson, Bioconjugate Techniques, San Diego: Academic Press, 1996). Interestingly, low modification (average of 1 to 3 PEG's per enzyme) of sarcosine oxidase with PEG- SPA caused a loss of less than 5% activity. At higher modifications (> 5 PEG chains attached) more significant loss of enzyme activity was apparent which could be prevented using the inhibitors pyrolecarboxylic and (methylthio)acetic acid. This indicates that the reactivity of PEG-SPA with residues on the surface of sarcosine oxidase is different than the reactivity of PEG-NCO with sarcosine oxidase.

Since modification of sarcosine oxidase at pH 7.5 caused a greater inactivation than at pH 8. 5, it seems likely that the modification of the terminal amine can responsible for the loss of activity since the terminal amine should be more reactive (the terminal amine has a lower pKa than the ÿ-amine of lysine). In fact, modification of sarcosine oxidase by PEG-NCO caused no noticeable shift in the visible absorption spectrum of sarcosine oxidase indicating that modification did not occur in the active site. The absorption spectrum of the enzyme-bound FAD of Corynebacterium sarcosine oxidase has been reported to change (red shift of absorption peak from 455 to 462nm) upon modification of histidine residue with diethylpyrocarbonate (DEP), probably in the vicinity of the flavin moiety, (Hayashi et al., J. Biochem. 94:551-558 (1983)). DEP-modified sarcosine oxidase is completely inactive; however, activity can be recovered by treatment with hydroxylamine. Although the absorption spectrum of Arthrobacter sarcosine oxidase did not shift upon modification with PEG-NCO, this is not absolute evidence that modification did not occur in the active site.

### Prediction of chemical modification site

In order to elcucidate the effect of chemical modification on sarcosine oxidase activity, computational studies were performed to predict the most important residues for enzyme activity/stability and the most reactive residues for modification.

The reactivity of acetylation on lysine residues in RNAse A as a function of the solvent accessibility (SA) of a residue and the pKa value of lysines has been described previously (Glocker et al., Bioconjugate Chem. 5:583-590 (1994)). Similar results have been shown for deoxy-hemoglobin (Scaloni et al., FEBS Letters 452:190-194 (1999)) and horseradish peroxidase (O' Brien et al., Biotechnol Bioeng 76:277-284 (2001)). Specifically, reactivity increases with increasing SA. However, even with low SA, the reactivity increases dramatically when the pKa of a given lysine residue is especially low (signifying a very good nucleophile). Hence, lysine residues located on the surface of the enzyme (with pKa∼10.5) will have a reactivity that is proportional to SA; however, residues located in an environment which can have a significant effect on pKa, like the active site lysine of bovine ribonuclease A, will have a significantly different pKa and altered reactivity.

The predicted pKa's for all lysine residues in sarcosine oxidase (FAD free-MSOX) are shown in Table 1.

**Table 1. Solvent accessibility and pKₐ predicted for the terminal amine and the lysine residues in sarcosine oxidase. Rows in bold show the residues located in or within six Angstroms of the active site of the enzyme.**

| Residue Number | Solvent Accessibility | pKₐ |
|---|---|---|
| MET 1 | 83.40 | 7.39 |
| LYS 4 | 43.22 | 10.66 |
| LYS 5 | 29.67 | 11.30 |
| LYS 27 | 30.40 | 12.22 |
| LYS 31 | 31.87 | 11.51 |
| LYS 80 | 58.97 | 11.38 |
| LYS 85 | 30.04 | 11.79 |
| LYS 89 | 34.07 | 10.72 |
| LYS 98 | 37.36 | 11.34 |
| LYS 112 | 54.95 | 11.22 |
| LYS 128 | 56.04 | 10.80 |
| LYS 145 | 36.63 | 11.07 |
| LYS 169 | 31.14 | 11.36 |
| LYS 187 | 20.88 | 10.69 |
| LYS 199 | 19.05 | 11.88 |
| lyes 210 | 29.30 | 13.04 |
| LYS 214 | 35.53 | 12.09 |
| LYS 236 | 57.51 | 10.61 |
| LYS 237 | 26.74 | 11.86 |
| **LYS 268** | **2.20** | **9.68** |
| LYS 277 | 48.72 | 11.38 |
| LYS 299 | 46.52 | 11.23 |
| LYS 313 | 38.10 | 12.12 |
| **LYS 322** | **24.18** | **13.67** |
| **LYS 351** | **4.76** | **9.18** |
| LYS 368 | 45.42 | 10.90 |
| LYS 384 | 45.42 | 10.86 |
| LYS 386 | 43.96 | 11.34 |

The SA was calculated from SWISS PDB viewer and estimated pKa was obtained from the UHBD algorithm. Based on these results, lysine 351 is the most reactive lysine since the pKa of this lysine is 9.18. Lysine 268 in the active site has the second lowest pKa of 9.68. The third active site lysine (lysine 322) has a significantly increased pKa (13.67) and is not suspected to be easily modified.

### Immobilization of sarcosine oxidase in polyurethane polymers

After finding the optimal conditions for modification of sarcosine oxidase to preserve enzyme activity retention during modification with isocyanate, the enzyme was immobilized in polyurethane hydrogels. Enzyme containing polymers that were prepared with enzyme concentrations of 0 to 200 U/g of polymer had a specific activity that was directly proportional to enzyme concentration (Figure 4). Since the reaction rate is proportional to enzyme concentration, this ensures that the rates measured under these conditions are not diffusion controlled and represent only the rate from the enzyme-catalyzed reaction (Yamane, Bioeng. 19:749-756 (1977)). Enzyme-polymers prepared without inhibitor retained no activity verifying that the effects seen with the soluble modifiers translated directly to the polyurethane biopolymer. Activity retentions of polymers prepared with inhibitor were approximately 10% of those found using the protected method.

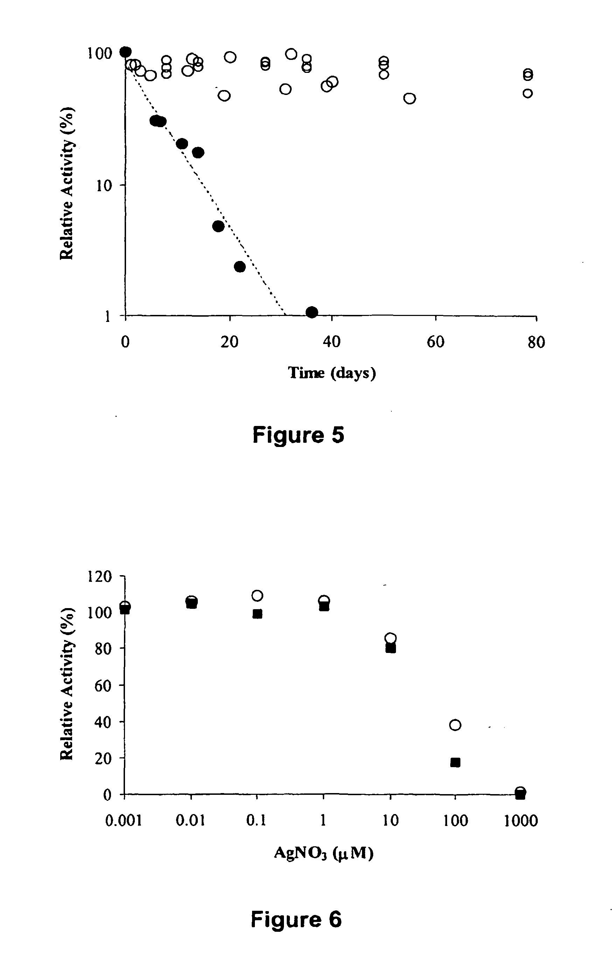

The immobilized polymers were tested for reusability by repeatedly assaying a single gel sample for eight cycles with no loss of apparent activity (Figure 5). As seen with other polyurethane chemistries, the enzyme is well immobilized within the gel and no loss of enzyme is observed (Lejeune and Russell, Biotechnol Bioeng 51:450-457 (1996); Lejeune et al., Biotechnol Bioeng 544:105-114 (1997); Drevon et al., Biotechnol Bioeng 79:785-794 (2002)). Polymers were also prepared and stored in buffer at 4 °C under gentle agitation. Samples were removed from the liquid phase to measure for enzyme activity that may have leached out. No activity was measured in the liquid phase over a nearly 50-day period (Figure 6). Since the enzyme is irreversibly immobilized in the polyurethane material , this immobilization technique will be ideal for use in reusable clinical biosensors.

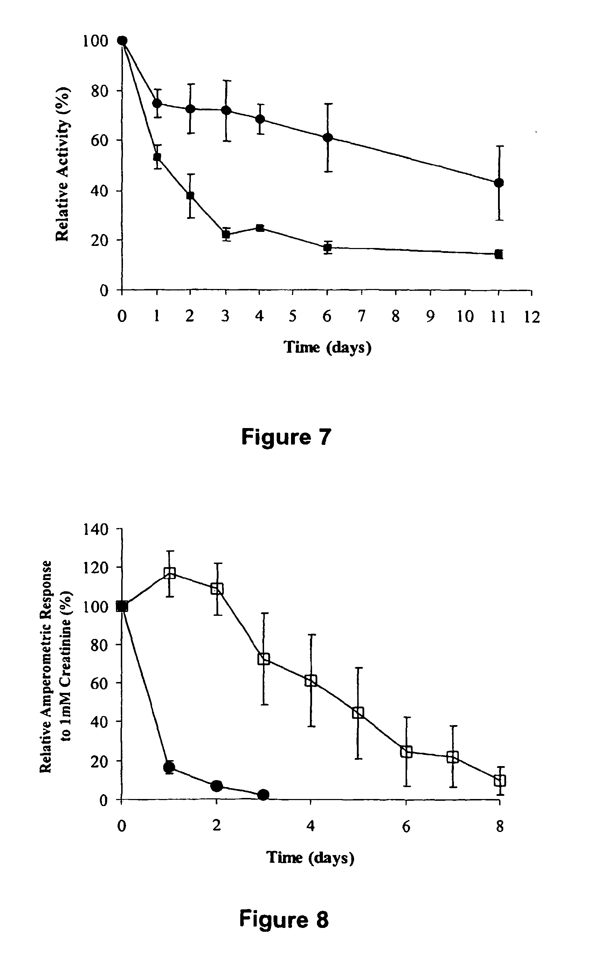

The thermoinactivation of the enzyme in the polymer was determined by incubating enzyme-polymer samples in buffer at 37 °C and removing samples for assay periodically (Figure 7). The half-life of native sarcosine oxidase at 37 °C is 7 days whereas immobilized sarcosine oxidase retained >50% activity after incubation at 37°C in buffer for more than 50 days. Obviously, inhibitor-protected immobilization of sarcosine oxidase in polyurethane polymers is effective in stabilizing the enzyme. High enzymatic stability, especially at elevated temperatures, will be essential for successful application in continuous use clinical blood analyzers.

### Effect of silver ions on enzyme activity

Since the enzyme will be applied to amperometric electrodes that contain silver/AgCl reference electrodes, the effect of silver ions on enzyme activity was explored. Inhibition of enzymes by silver ions from reference electrodes has been noted before (Schaffar, Anal Bioanal Chem 372:254-260 (2002); United States Patent No. 4,547,280) and should be adequately assessed since silver ions can bind very tightly to an enzyme and lead to deactivation.

In order to test sarcosine oxidase for inhibition by silver ions, 1 nM to 1 mM silver nitrate was added to a solution of enzyme and incubated for various amounts of time. The enzyme was then removed from solution and assayed for residual sarcosine oxidase activity in the absence of silver (Figure 8). Obviously, silver is an effective irreversible inhibitor of sarcosine oxidase. The enzyme inhibition appears to be slow requiring long incubation times to see an effect at low silver concentrations. Although the inhibition may not be instantaneous, since these sensors can be used over a period of a couple of weeks, inhibition by silver can shorten sensor half-lives and strategies to diminish the impact of silver ions on biocatalytst-based sensors can be employed as described above to diminish or prevent inavtivation.

### EXAMPLES II

### MODIFICATION AND IMMOBILIZATION OF CREATINE AMIDINOHYDROLASE IN A POLYMERIC SENSOR ENVIRONMENT

This example describes the immobilization and stabilization of creatine amidinohydrolase modified with isocyanate activated polyethylene glycol (PEG).

### A. Materials and Protocols

Creatine amidinohydrolase (from Actinobacilus sp., CRH-211) and sarcosine oxidase (from Arthrobacter sp., SAO-341) were purchased from Toyobo Co., LTD. All enzymes were used without further purification. PEG-NCO (Mw 5 000) was obtained from Shearwater Polymers Inc. (Huntsville, AL). Hypol 2060G prepolymer was purchased from Hampshire Chemical (Lexington, MA). All other reagents were purchased from Sigma-Aldrich Chemicals (St. Louis, MO) and were of the highest purity available.

### PEGylation of Creatine Amidinohydrolase

PEG-NCO was added at room temperature to a buffered solution (50 mM phosphate, pH 7. 5) containing 3 mg/mL creatine amidinohydrolase. The ratio PEG-NCO/Enzyme was adjusted from 0/1 to 100/1. The reaction was mixed for 30 min followed by overnight dialysis against 50 mM phosphate buffer at 4 °C (Drevon et al., Biomacromolecules 2:764-771 (2001)). Enzyme activity following modification was determined using an end point assay (described below).

### Synthesis of Creatine Amidinohydrolase-Containing Polyurethane Hydrogel

Hypol prepolymer 2060G (0.4 g), a toluene diisocyanate based prepolymer, was added to a buffered solution (3. 6 g of 50 mM phosphate buffer, pH 7.5) containing creatine amidinohydrolase (0 to 100 units enzyme per gram of pre polymer). The aqueous polymer solution was mixed for 30 s in a weigh boat until the onset of gelation. Polymerization was very rapid and gelation was usually complete within one minute.

### Characterization of Enzyme Modification

MALDI-MS analyses were performed using a Perspective Biosystems Voyager Elite MALDI- TOF. The acceleration voltage was set to 20 kV in a linear mode. 1 µL of PEGylated enzyme solution (0. 1 mg/mL) was mixed with I µL of matrix solution (0.5 mL water, 0.5 mL of acetonitrile, 1 µL of trifluoracetic acid, and 10 mg of sinapinic acid). Spectra were recorded after evaporation of the solvent mixture and were calibrated externally using equine cytochrome C (12,361.96 Da), rabbit muscle aldolase (39,212.28 Da) and bovine serum albumin (66 430.09 Da).

### End Point Assay for Creatine Amidinohydrolase Activity

Creatine amidinohydrolase activity was monitored using a colorimetric assay that measures urea formation from the hydrolysis of creatine (Tsuchida and Yoda, Clin. Chem. 29:51-55 (1983)). An enzyme solution (0.1 mL) was incubated with a mixture (0.90 mL) of 100 mM creatine in sodium phosphate buffer (50 mM, pH 7.5) at 37 "C for 10 minutes. The reaction was stopped by adding 2.0 mL of a solution containing Ehrlich's reagent (2.0 g of p-dimethyl aminobenzaldehyde in 100 mL of dimethyl sulfoxide plus 15 mL of concentrated HCl). The solution was incubated for 20 minutes at room temperature and the absorbance at 435 nm was measured. One unit was defined as the amount of enzyme that catalyzes the hydrolysis of 1 µmol of substrate per minute.

### Oxygen Monitor Assay for Creatine Amidinohydrolase Activity

The initial rate of oxygen consumption was measured at 37 °C with a Clark oxygen electrode from Yellow Springs Instruments (Yellow Springs, OH). The reaction was initiated by adding an enzyme solution (1 µL) or an enzyme-containing polymer (10-100 mg cut into small pieces) to 5.0 mL of substrate (100 mM creatine in 50 mM phosphate buffer, pH 7. 5 with at least 6 U/mL sarcosine oxidase). Before measurement the assay solution was allowed to equilibrate to 3.7 °C with air. Oxygen consumption was measured for 5 to 10 minutes.

### Thermostabilty of Native and PEG-Modified Creatine Amidinohydrolase

Thermoinactivation of native and PEG-creatine amidinohydrolase was monitored at 37 °C in buffer (50 mM sodium phosphate, 2 mM EDTA, pH 7.5). The enzyme concentration in all samples was 0.08 to 0. mg/ml. Samples were removed periodically and assayed for activity using the end point assay.

### Thermostabilty of Immobilized Creatine Amidinohydrolase

Enzyme polymer samples were cut into small pieces and added to buffer (50 mM phosphate, pH 7. 5) and incubated at 37 °C. Samples were removed periodically and assayed for enzymatic activity using the oxygen electrode.

### Silver Inhibition of Creatine Amidinohydrolase

Creatine amidinohydrolase (1.0 mg/mL) was incubated in 20 mM Tris-HCl (pH7. 5) with silver nitrate (0 to 1 mM) at room temperature. Samples were removed periodically and assayed using the end point assay. In order to test if creatine acted as a competitive inhibitor for silver inhibition, the end point assay was performed as above with silver nitrate added to the creatine solution (0 to 100 µM AgN03 in 20 mM Tris buffer). After 10 minutes of incubation, the stop solution (p-dimethyl benzaldehyde solution) was added. Control experiments showed silver nitrate had no effect on the color development.

### Use of Additives to Prevent Silver Inhibition of Creatine Amidinohydrolase

Creatine amidinohydrolase (1.0 mg/ml) was prepared in solution with either 50 mM EDT A, 50 mM EGT A, 50 mM mercaptoethanol, 50 mM DTT or 50 mM cysteine with 20 mM Tris, pH 7. 5. Silver nitrate was added to give a final concentration of 0 to 100 µM. After the addition of silver nitrate, the solutions were allowed to incubate for 5 minutes and residual activity was determined using the end point assay.

### UV- Vis Absorption Spectra of Creatine Amidinohydrolase

UV spectra were determined as described by Shen et al., J. Inorg. Biochem, 95:124-130 (2003). In short, creatine amidinohydrolase (1 mg/mL) was dissolved in 20 mM Tris (pH 7.5) with varying concentrations of silver nitrate (0-100 µM). In quartz cuvettes, the UV absorption spectra of the enzyme solutions were measured from 230 to 300 nm. Difference spectra were obtained by subtracting the spectrum of the native enzyme (no silver) from the silver-enzyme spectra.

### Selection of a Suitable Template for GNM Analysis.

The crystal structure of Actinobacillus creatinase as described by Padmanabhan et al., Acta Crystallogr., Sect. D 58(8): 1322-1328 (2002) (PDB code: 1KP0) was used for Gaussian Network Model (GNM) (Bahar et al., Folding Design 2:173-181 (1997)) analysis of collective dynamics. The CMS (creatine analog) bound A.Bacillus creatinase structure was computationally modeled by combining the CMS molecule in P.putida creatinase strcture (PDB code:1CHM) with the A.Bacillus creatinase structure, followed by standard energy minimization using the MOE package (Molecular Operating Environment, world wide web at chemcomp.com/Corporate_Infonnation/MOE_Bioinfonnatics.html).

### B. Results

### Effect of PEGylation on Enzyme Activity and Stabilty

With regard to the effect of chemical modification on creatine amidinohydrolase activity and stability, PEGylation accurately mimics the first steps in polyurethane-based immobilization strategies. Therefore, the enzyme was PEGylated. Creatine amidinohydrolase can be modified with reactive PEG's to a high degree without significant loss of enzyme activity. Modification of each monomer of the enzyme with an average of 5 PEG chains led to loss of only 30 % activity.

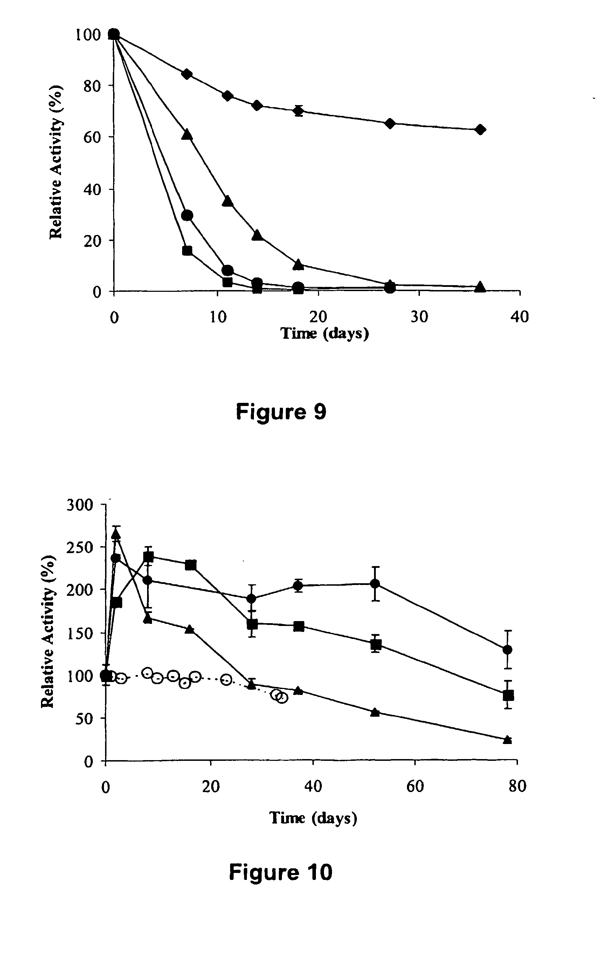

Although the majority of enzyme activity was retained after modification, a significant decrease in enzyme stability was observed when the modified-enzyme was stored in buffer at 37 °C (Figure 9). Native enzyme loses less than 40 % activity in buffer at 37 °C over 40 days; however, the modified enzyme showed a substantial decrease in stability in solution. Since creatine amidinohydrolase is a homodimer with a low intrinsic stability (Schumann et al., Biol. Chem. 374:427-434 (1993)) it is possible that chemical modification modifies the protein in such a way that it is more easily unfolded. Although PEGylation often predicts the impact of polyurethane immobilization on activity, the stabilization of proteins by immobilization can be significantly different than the impact of PEGylation.

### Immobilization of Creatine Amidinohydrolase in Polyurethane Polymers

Biopolymers were prepared with enzyme concentrations of 0 to 100 units/g of polymer. The specific activity of the enzyme-polymers was directly proportional to enzyme concentration (Figure 10). This implies that the rates measured under these conditions are not diffusion-controlled and reflect only the enzymatic reaction rate (Yamane, Biotechnol. Bioeng. 19:749-756 (1977)). The average activity retention for creatine amidinohydrolase in the polyurethane polymers was 28 %.

The immobilized polymers were tested for reusability by repeatedly assaying a single gel sample for eight cycles with no loss of apparent activity (Table I). As seen with other polyurethane chemistries, the enzyme is well immobilized within the gel and no leaching of enzyme is observed (Lejeune and Russell, Biotechnol Bioeng 51:450-457 (1996); Lejeune et al., Biotechnol Bioeng 54:105-114 (1997); Drevon et al., Biotechnol Bioeng 79:785-794 (2002)).

The storage stability of the enzyme in the polymer was determined by incubating enzyme-polymer samples in buffer at 37 °C and removing samples periodically to be assayed (Figure 10). Interestingly, polymerized enzyme reproducibly increased observed activity during the first week of storage. This increase in activity can be due to a change in polymer properties; however, this type of effect was not observed with other enzymes in these polymers. After the first week, the enzyme activity began to decrease and the rate of loss in observed enzyme activity was dependent on the concentration of the enzyme in the polymer. Polymers with more enzyme showed slower deactivation. Nonetheless, significant activity was retained in all polymers for up to 80 days which is more than long enough for use in a diagnostic biosensor. Obviously, the stability reduction caused by PEGylation was not observed in the immobilized enzyme. Given that only upon immobilization is the enzyme "locked" into a fixed conformation these data support an intuitive expectation of stability enhancements.

### Effect of Silver on Enzyme Activity

Before being used in a functioning sensor, polyurethane-immobilized enzyme must be applied to amperometric electrodes that contain Ag/ AgCl. Given the known propensity of silver ions to interact with proteins (Schaffar, Anal Bioanal Chem 372:254-260 (2002); United States Patent No. 4,547,280), the effect of silver ions on enzyme activity was explored. Silver-induced inactivation becomes more of a concern if a sensor will be used in series with other sensors for a long period of time since the enzyme will have significant time to scavenge silver from solution.

In order to test creatine amidinohydrolase for inhibition by silver ions, the enzyme was incubated with 5-100 µM silver nitrate and assayed for residual creatine amidinohydrolase activity. Silver is a very effective inhibitor of creatine amidinohydrolase completely inhibiting enzyme activity. Furthermore low concentrations of silver effectively inactivate the enzyme; in fact, the silver concentration is on the same order as the enzyme concentration.

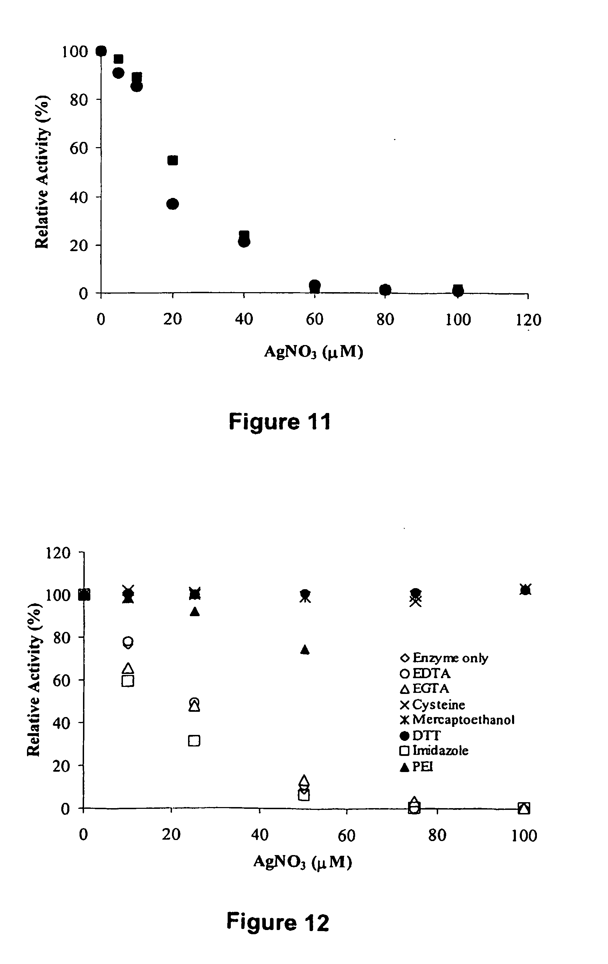

In order to determine whether silver was acting as a competitive inhibitor differing concentrations of silver and 90 mM creatine were added to the buffer. The enzyme (∼7 µM) was added directly to this solution and after 10 min the concentration of urea in the system was determined (Figure 11). The presence of creatine did not slow enzyme inhibition. Silver is likely binding at a location other than the active site; otherwise, its inhibitory activity would be reduced by the presence of high concentrations of the substrate. It is also important to note that the inactivation was not reversible. Dilution of the inhibited enzyme did not regenerate activity; furthermore, dialysis overnight with either EDT A or DTT did not reactivate the enzyme. This indicates that silver is bound very tightly to the enzyme and is not likely to dissociate.

Since the enzyme is effectively inhibited by silver ions at concentrations that could be released from electrodes in a clinical biosensor, it is of interest to develop methods to prevent or at least slow enzyme inhibition by silver: Silver can be effectively scavenged by a number of molecules. The use of 50 mM solutions of EDTA, EGTA, DTT, mercaptoethanol, cysteine, imidazole and polyethyleneimine (PEI) was employed to sequester silver. Inhibition by silver ions was effectively prevented by pre-incubation with thiol containing compounds (cysteine, mercaptoethanol, DTT) and prevented somewhat by PEI (Figure 12). Hence, thiol-containing compounds can be effective in scavenging silver ions that leach from the electrode and can be useful in preventing enzyme deactivation.

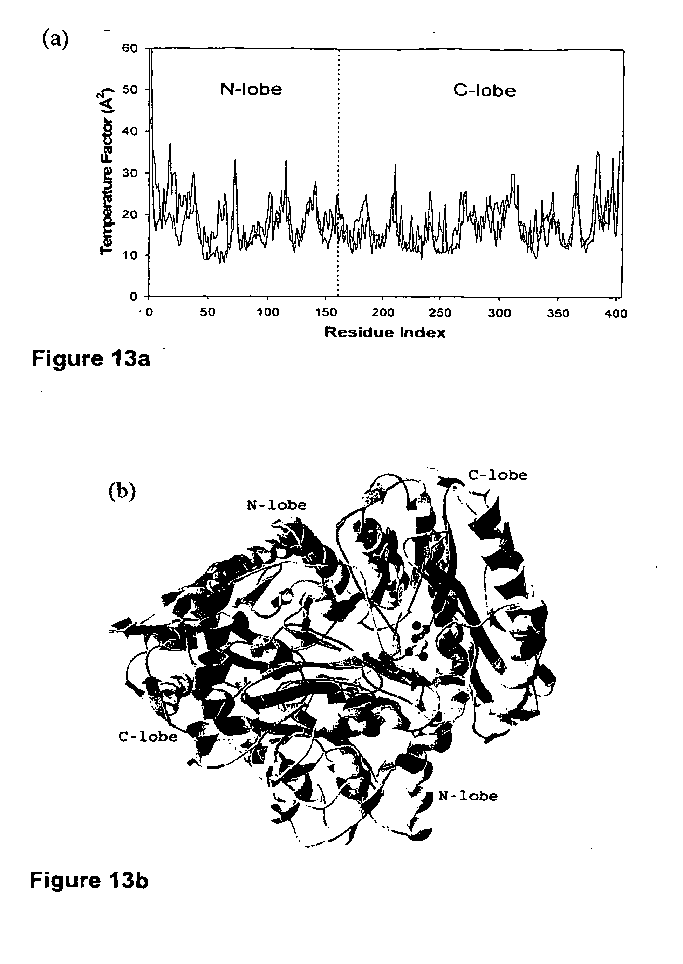

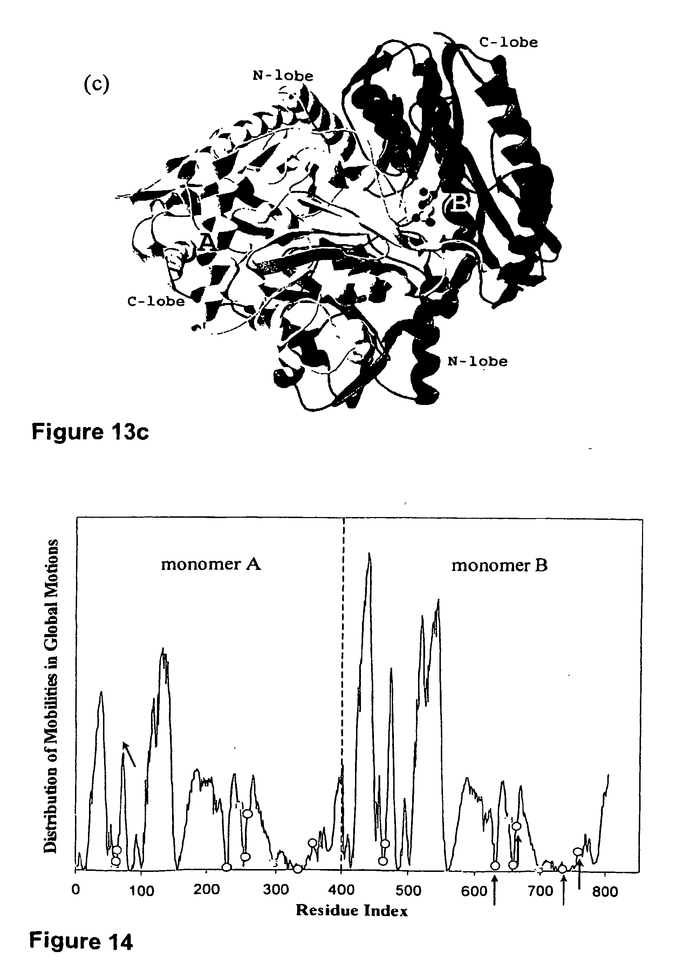

UV/Vis spectroscopy was used to determine if any major structural changes occurred upon binding of silver to the enzyme (Figure 13). An increase in absorbance around 247 nm is apparent with increasing concentrations of silver. This observed peak is likely due to the formation of ligand-to-metal charge transition (LMCT) bands. The LMCT bands are due to charge transitions that occur when ligands in creatine amidinohydrolase bind to the metal center of Ag (I) (Shen et al., J Inorg Biochem 95:124-130 (2003)). Raman spectroscopy has been used to show that silver ions form covalent bonds with sulfur atoms in human serum albumin (HSA) (Shen et al., supra, 2003).. Since Ag (I) is a soft Lewis acid, it should have a high affinity for the soft donor sulfur atom (ref) and hence one can expect strong interactions between the cysteine residues of creatine amidinohydrolase and silver.

Modification of sulfhydryl groups on creatine amidinohydrolase causes complete loss of activity (Coll et al., J Mol Biol 214:597-610 (1990) and Yoshimoto et al., J Biochem 79:1381-1383 (1976). This is interesting since the Cys residues are all far from the active site and have no known role in catalysis (Coll et al., supra, 1990; Hoeffken et al., J Mol Biol 204:417-433 (1988)). Alkylation of Cys298 inactivates the enzyme even though this residue is far from the active site and the loss of activity can be attributed to the possible prevention of domain motions by alkylation, thus locking the enzyme into a given conformation.

### Collective dynamics of the enzyme and important role of cysteine residues

To further investigate how Cys residues, and their interaction with silver ions, might contribute to the function and stability of creatine amidinohydrolase, the dynamics of the protein were examined with the Gaussian Network Model (GNM) (Bahar et al., supra, 1997). The GNM is an elastic network model that has been shown to predict the collective dynamics of proteins, in close agreement with the temperature (B-) factors from X-ray crystallographic experiments (Bahar, Folding Design 2:173-181 (1997); Kundu et al. Biophys. J. 83: 723-732 (2002)), as well as the free energy costs of H/D exchange (Bahar et al., Biochemistry 37:1067-1075 (1998b)). The approach permits decomposition of the conformational motions into a series of orthogonal modes, ranked by their associated frequencies. The modes with the slowest frequency (the slowest mode), also referred to as the 'global' motions, usually reveal the functional movements that engage the entire molecule (Kitao and Go, Curr. Op. Struc. Biol. 9(2): 164-169 (1999); Ming et al., Procl. Nat. Acad. Sci. USA 99:8620-8625 (2002);Haliloglu and Bahar, Proteins: Structure, Function and Genetics 37:654-667 (1999); Bahar and Jernigan, J. Mol. Biol. 281:871-884 (1998); Bahar et al., Phys. Rev. Lett. 80:2733-2736 (1998); Bahar et al., J. Mol. Biol. 285:102301037 (1999); Tama and Sanejouand, Protein Engineering 14:1-6 (2001)). The fastest modes, on the other hand, indicate the 'local' motions , and point to individual residues inyolved in the early folding/stabilization process (Bahar et al. Phys. Rev. Lett. 80:2733-2736 (1998); Rader and Bahar, Polymer 45(2):659-668 (2004). The major utility of the GNM is its effcient applicabilty to large structures' dynamics (such as creatine amidinohydrolase, a dimer of N = 804 residues) that are beyond the range of molecular dynamics simulations.

As a first test, the mean-square fluctuations of creatine amidinohydrolase residues predicted by the GNM were compared with the experimental B factors (Padmanabhan et al., supra, 2002) (Figure 13(A)). The results are displayed for only one monomer since both monomers show almost identical fluctuation behavior. The monomer structure is composed of two lobes, N-lobe and C-lobe at the N- and C-termini composed of 160 and 240 residues, respectively. The N-terminal end shows the highest fluctuations. The close agreement between theoretical and experimental values lends support to further examination of collective motions with the GNM. Panel b displays the ribbon diagram of the enzyme, color-coded from red to blue, in the order of decreasing mean-square-fluctuations. Panel C shows the location of the two monomers and the substrate analog (CMS).

Next, global modes of motions were used to infer information on functional dynamics. Figure 14 shows the mobilities of residues in the most representative global mode of the enzyme, which effectively displays the demarcation between the two lobes. The ordinate scales with the square displacements of the individual residues. Peaks indicate the most mobile regions, and minima refer to the global hinge (or anchoring) regions, about which the concerted motions of large substructures (domains, subunits, etc) take place. The residues involved in the catalytic activity of the enzyme (shown by the open blue circles) exhibit minimal fluctuations. This type of mechanical constraint near the catalytic site is consistent with the fine tuning and cooperativity of enzymatic activity (Barlett and Thornton, J. Mol. Biol. 324:105-121 (2002)). Notably, the substrate analog (CMS) is not symmetrically bound with respect to the two monomers in the PDB structure, but interacts more closely with one of the chains (monomer B) and subset of catalytic residues (shown by arrows in Figure 14), although both chains exhibit the same global dynamics, and thus are equally disposed to bind the substrate.

Panel (B) in Figure 15 displays the same results in the color-coded ribbon diagram of monomer B. Using the same convention as in Figure 14, the mobile regions are colored red , and the most severely constrained regions are colored blue. Regions of intermediate mobility are colored orange-yellow-green-cyan, in the order of decreasing mobility. The terminal lobe exhibits the largest amplitude motions. Cys60 and Cys297 are both located in highly constrained hinge/anchoring regions on the respective C- and N-lobes (Figure 15(A)). In particular Cys297 resides in a highly stable central region near the interface between the two lobes, and plays a dual role in controlling domain and subdomain motions (evidenced by the minima observed at this residue in the dominant modes). The N-terminal domain serves as a concertedly moving lid that allows the cat.alytic pocket in the C-terminal domain of the other chain to be exposed to solvent and to recruit the substrate inside. Cys60 belonging to the A chain closely coordinates the substrate, and is also distinguished by severely constrained and highly cooperative dynamics (minima in panel A)

Figure 16 provides a closer view of the catalytic binding pocket. The oxygen atoms 0ε1 of Glu357 and 0 ε2 of Glu26 1 on monomer B form hydrogen bonds with a nitrogen atom in the guanidine group of CMS (Figure 16), which can facilitate the peptide bond breakage between the guanidine group and the resultant sarcosine molecule. Likewise, the residues Cys60, Phe62 and-Arg64 on monomer A are spatially close to the B-monomer His231, the most critical residue that dictates the catalytic chemistry of creatine (Coll et al., J. Mol. Biol. 214:597-610 (1990); Padmanabhan et al., supra, 2002). His231 forms three hydrogen bonds with the substrate (shown by dashed lines), but not with any other amino acid. The sulfide atom (Sy) on Cys60 could form a 'hydrogen-bond like' interaction with the nitrogen Nδ1 on His 231 , which could assist His231 side chain to stabilize in a catalytically potent conformation.

The folding nuclei were inferred from the high frequency modes predicted by the GNM. Among the three cysteine residues, Cys297 is distinguished by a peak in Figure 16. Cys297 is known to be of the most critical residues that are involved in the early folding process in creatine amidinohydrolase. A a peak near Cys249 also was observed. Cys249 is located in the folding core composed of three β-sheet (β9, (β11 and β12) and part of two α-helices (α7 and α8) in a pita bread fold of C-terminal lobe yet has only minor structure significance in the folding process. Moreover from the electrostatic analysis, it was found that Cys297 is surrounded by negatively charged patches. Those patches are suspected to preferentially recruit silver ion to Cys297, which was found both structurally and functionally critical, rather than other cysteines. One intuitive approach to reduce silver sensitivity of the creatine amidinohydrolase could be to mutate the residues responsible for the negative patches around Cys297. Also, single mutation of Cys60 to Ser or Thr should preserve the functional character of Cys60 while abating potential silver ion attack.

Significantly, silver binds strongly with creatine amidinohydrolase and completely inhibits enzyme function even at low concentrations. As reported in the other papers in this series, all three enzymes needed for the creatinine biosensor are inhibited to some extent by silver; thus, methods for preventing or slowing enzyme inactivation by silver are required.

### EXAMPLE III

### MODIFICATIONS AND IMMOBILIZATION OF CREATININE AMIDOHYDROLASE USING POLYURETHANE PREPOLYMERS

This example describes the chemical modification and immobilization of the enzyme creatinine amidohydrolase into polyurethane prepolymers.

### A. Materials and Protocol

Creatinine amidohydrolase (from microorganism CNH-311), creatine amidinohydrolase (from *Actinobacilus* sp., CRH-211) and sarcosine oxidase (from *Arthrobacter* sp., SAO-341) were purchased from Toyobo Co., LTD. All enzymes were used without further purification. PEG-SPA (Mw 5000) was obtained from Shearwater Polymers Inc. (Huntsville, AL). Hypol 20600 prepolymer was purchased from Hampshire Chemical (Lexington, MA). All other reagents were purchased from Sigma-Aldrich Chemicals (St. Louis, MO) and were of the highest purity available.

### PEGylation of Creatinine Amidohydrolase

PEG-SPA was added at room temperature to a buffered solution (50 mM phosphate, pH 7.5) containing 3 mg/mL creatine amidinohydrolase. The ratio PEG..SPA to Enzyme- was adjusted from 0/1 to 100/1. The reaction mixture was mixed for 30 min followed by overnight dialysis (12 000 MWCO) against 50 mM phosphate buffer at 4 °C (Drevon et al., Biomacromolecules 2:764-77 (2001)). Enzyme activity following modification was determined using the end point assay.

### Synthesis of Creatinine Amidohydrolase-Containin Polyurethane

Hypol prepolymer 2060G (0.4 g), a toluene diisocyanate based prepolymer, was added to a buffered solution (3.6 g of 50 mM phosphate buffer, pH 7.5) containing creatinine amidohydrolase (0 to 150 units enzyme per gram of prepolymer). The aqueous polymer solution was vigourously mixed for 30 s, using a spatula, in a weigh boat until the onset of gelation. Polymerization was very rapid and gelation was usually complete within one minute.

### Characterization of Enzyme Modifcation

MALDI-MS analyses were performed using a Perspective Biosystems Voyager Elite MALDI-TOF. The acceleration voltage was set to 20 kV in a linear mode. 1 µL of PEGylated enzyme solution (0.1 mg/mL) was mixed with 1 µL of matrix solution (0. mL water, 0.5 mL of acetonitrile, 1 µL of trifluoracetic acid, and 10 mg of sinapinic acid). Spectra were recorded after evaporation of the solvent mixture and were calibrated externally using equine cytochrome c (12 361.96 Da (ave)), rabbit muscle aldolase (39,212.28 Da (ave)) and bovine serum albumin (66,430.09 Da (ave)).

### Measurement of Creatinine Amidohydrolase Activity using an End Point Assay

The measurement of creatinine amidohydrolase activity is based on the Jaffé reaction (Tsuchida and Yoda, Clin. Chem. 29:51-55 (1983)). An enzyme solution (0.1 mL) was incubated with a mixture (0.90 mL) of 100 mM creatine in mM soditim phosphate (pH 7.5) at 37 °C for 10 minutes. After 10 minutes, the creatine/enzyme mixture (0.1 mL) was added to 0.5 M sodium hydroxide (1.9 mL) and 1% picric acid (1.0 mL). This solution was incubated for 20 minutes at room temperature and the absorbance at 520 nm was measured. One unit was defined as the amount of enzyme which catalyzed the formation of 1 µmol of creatinine per minute.

### Measurement of Creatinine Amidohydrolase Activity using an Oxygen MonitorAssav

The initial rate of oxygen consumption was measured at 37 °C with a Clark oxygen electrode from Yellow Springs Instruments (Yellow Springs, OH). The reaction was initiated by adding an enzyme solution (1 µL) or an enzyme-containing polymer (10-100 mg cut into small pieces) to 5.0 mL of substrate (1.0 mM creatinine in 50 mM phosphate buffer, pH 7.5 with at least 18 U/mL sarcosine oxidase and 10 U/mL creatine amidinohydrolase). Before measurement, the assay solution was allowed to equilibrate to 37 °C with air. Oxygen consumption was measured for 5 to 10 minutes.

### Thermostability of Native and PEG-Modified Creatinine Amidohydrolase

Thermoinactivation of native and PEG-creatine amidinohydrolase was monitored at 37 °C in buffer (50 mM sodium phosphate, 2 mM EDTA, pH 7.5). The enzyme concentration in all samples was 0.01 to 0.02 mg/ml. Samples were removed periodically and assayed for activity using the end point assay.

### Thermostability of Immobilized Creatinine Amidohydrolase

- Enzyme polymer samples were cut into small pieces and added to buffer (50 mM phosphate, pH 7.5) and incubated at 37°C. Samples were removed over time and assayed for enzymatic activity using the oxygen electrode.

### Silver Inhibition of Creatinine Amidohydrolase

To determine the effect of silver ions on creatinine amidohydrolase, 57 µg/mL of lyophilized enzyme was incubated in 20 mM Tris-HCl (pH 7.5) with silver nitrate (0 to mM) at room temperature. Samples were removed periodically and assayed using the end point assay.

### Effect of Sensor Chips on Immobilized Enzyme Stability

The relative activities of three-enzyme containing polyurethane membranes were tested by storing membranes (20 mg hydrated) in 1 mL of 50 mM phosphate buffer (pH 5) with (two planar sensors) and without planar sensors in 5 mL Wheaton vials. The membranes were removed and assayed using the oxygen monitor with 1 mM creatinine. The rate of oxygen consumption was measured and the membranes were replaced to be re-assayed the following day. The thee enzyme polyurethane membranes were prepared using 1000 units/g prepolymer of sarcosine oxidase and creatine amidinohydrolase and 2500 units/g prepolymer of creatinine amidinohydrolase in 50 mM phophate buffer (pH 7.8) with 50 mM pyrrole-2-carboxylic acid.

### Prevention of Silver Inhibition in Biosensor

Creatinine biosensors were prepared by casting an enzyme-containing polyurethane membrane layer onto an amperometric sensor chip to give a wet polymer thickness of approximately 25 micrometers. Sensor chips were placed into a sensor body and tested using a stop flow setup. Hydrogen peroxide produced from the enzymatic degradation of creatinine was measured amperometrically using a BAS Voltammograph CV-37. Baseline readings were measured using 50 mM phosphate buffer (pH 7.5). The response to 1mM creatinine was determined by injecting approximately 1 mL of creatinine solution though the sensor body. The increase in amperometric response after 2 minutes was measured. When sensors were not being tested, they were stored at 37 °C in 50 mM phosphate buffer. In order to prevent silver chloride from leaching from the reference electrodes, some sensors were prepared by spotting the electrode surfaces with a cellulose acetate solution (5 wt% in acetone) and were allowed to air dry before application of the enzyme-membrane layer.

### B. Results

### Effect of PEGylation on Enzyme Activity and Stability

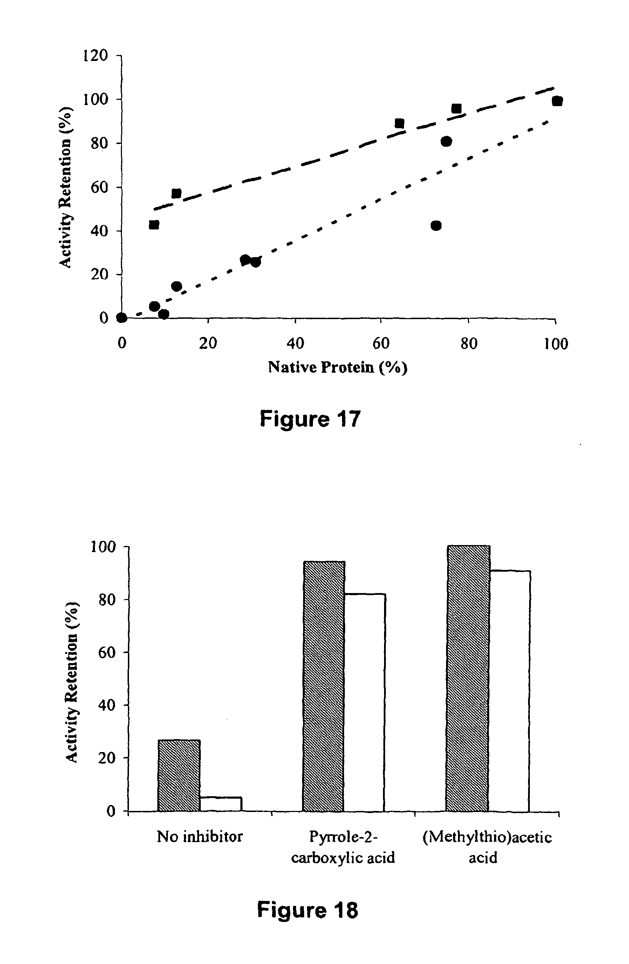

In order to determine the effect of chemical modification on creatinine amidohydrolase activity and stability, PEGylation studies were performed. PEG-modifications were made using an NHS ester of PEG (PEG-SPA) at different ratios of PEG-SPA to lyophilized enzyme powder. The enzyme could easily be modified by the PEG-SP A (Figure 17) with a loss of only 30% of activity with the attachment of an average of 5 PEGs per molecule of enzyme.

The enzyme stability in buffer at 37 °C was observed after modification (Figure 18). Very little difference between the native and modified enzyme was observed in solution at 37 °C. The native enzyme half-life of 6 days at 37 °C was reduced to 2 days if 2 mM EDTA was included in the solution. Since creatinine amidohydrolase is the most unstable enzyme tested, at pH 7.5 and 37 °C, strategies can be employed to stabilize the enzyme.

### Immobilization of Creatinine Amidohydrolase in Polyurethane Polymers

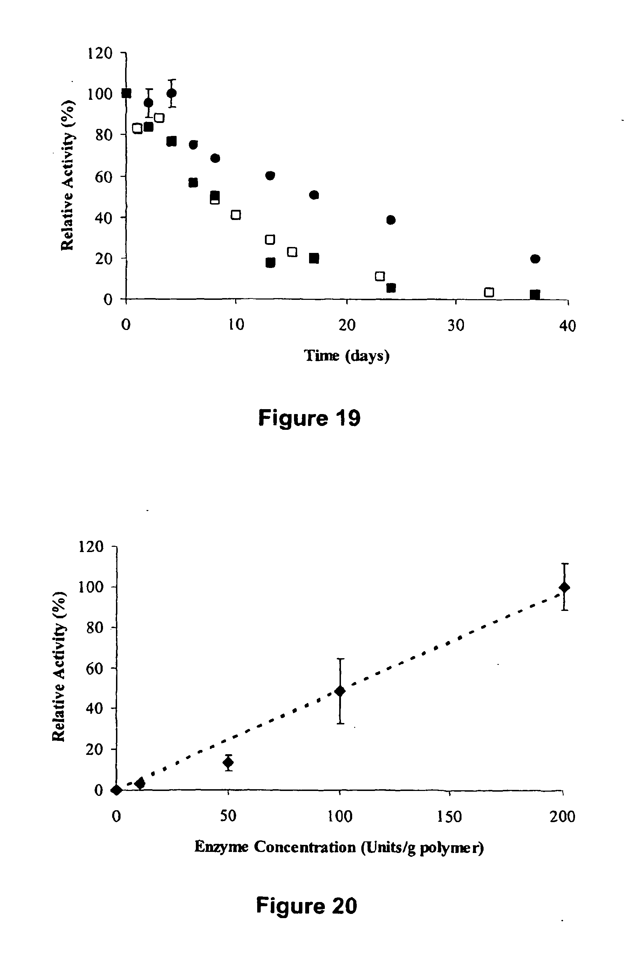

Polymers were prepared with enzyme concentrations of 0 to 150 U/g of polymer and showed a linear increase of specific activity with increase in enzyme concentration (Figure 19). This ensures that the rates measured under these conditions are not diffusionally controlled and reflect only the enzymatic reaction rate (Yamane, 1977).

The immobilized polymers were tested for reusability by repeatedly assaying a single gel sample for eight cycles with no loss of apparent activity (Figure 20). As seen with other polyurethane chemistries, the enzyme is well immobilized within the gel and no loss of enzyme is observed (Lejeune and Russell, Biotechnol Bioeng 51:450-457 (1996); Lejeune et al., Biotechnol Bioeng 54:105-114 (1997); Drevon et al., Biotechnol Bioeng 79:785-794 (2002)).

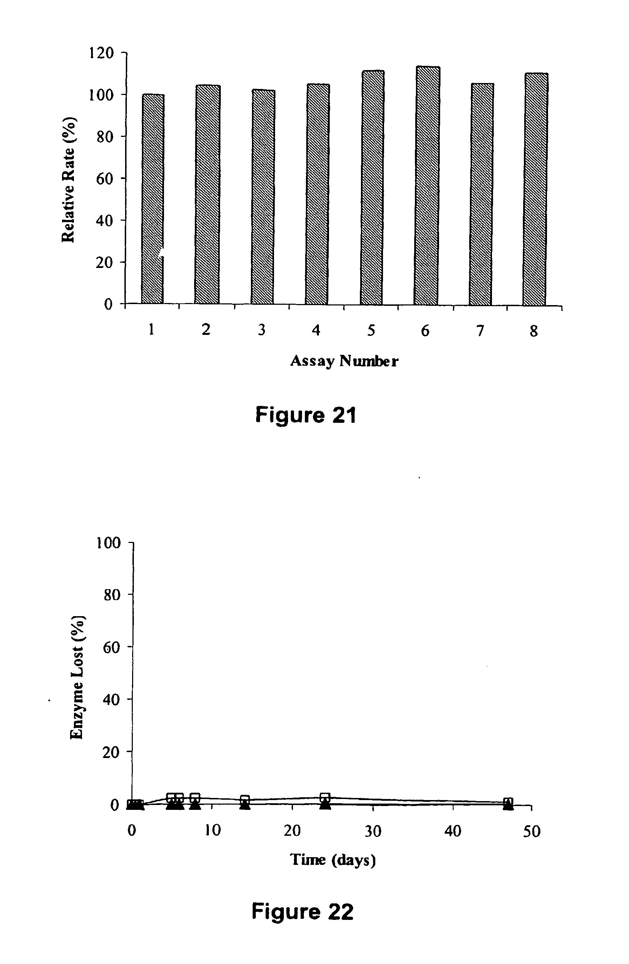

The thermoinactivation of the enzyme in the polymer was determined by incubating enzyme-polymer samples in buffer at 37 °C and removing samples for assay periodically (Figure 21). Native enzyme quickly loses activity in buffer at 37 °C with a half-life of 6 days. However, after 90 days at 37 °C in buffer, the immobilized enzyme exhibited greater than 50% activity retention. This significant increase in activity retention will be essential to ensure increased half-lives of the creatinine biosensors.

### Effect of Silver on Enzyme Activity

Since the enzyme will be applied to amperometric electrodes that contain Ag/AgCl, the effect of silver ions on enzyme activity was explored. Inhibition of enzymes by silver ions from reference electrodes has been noted before (Schaffar, Anal Bioanal Chem 372:254-260 (2002); United States Patent No. 4,547,280) and should be adequately assessed since silver ions can bind very tightly to the enzyme causing loss in activity. This becomes more of a concern if a sensor will be used in series with other sensors for a long period of time since the enzyme will have significant time to scavenge silver from solution.

In order to test creatinine amidohydrolase for inhibition by silver ions, 1 nM to mM silver nitrate was added to a solution of enzyme (<2 µM) and incubated for various amounts of time. The enzyme was then removed from solution and assayed for residual creatinine amidinohydrolase activity (Figure 22). Obviously, silver is an effective inhibitor of creatinine amidinohydrolase at high concentrations. The enzyme inhibition appears to be immediate with little change in impact from a five minute incubation to a one hour incubation time.

### Stability of Three-Enzyme-Containing Polymers in Solution

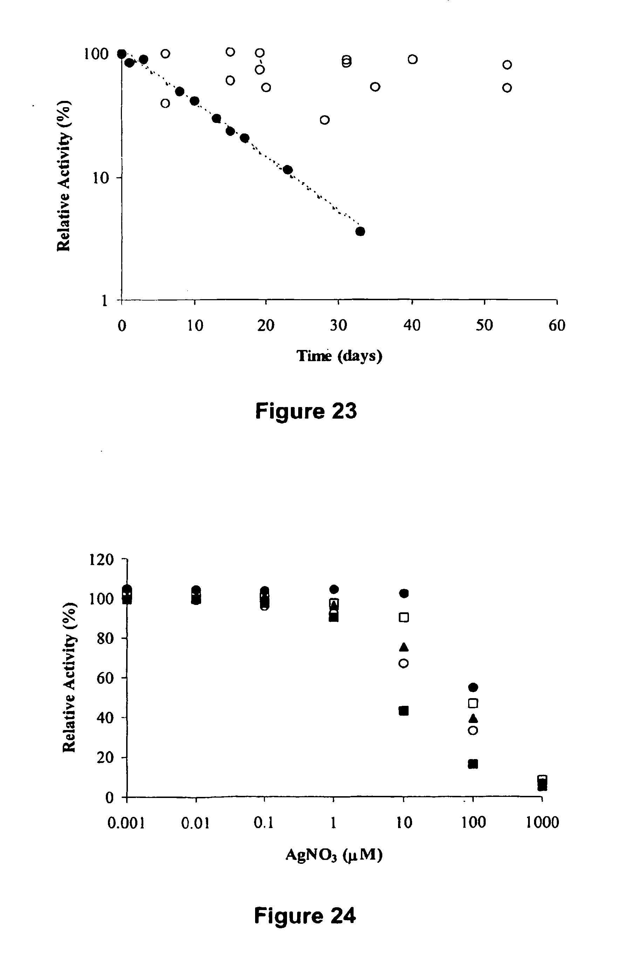

In order to test the Stability of the three-enzyme-containing polymer (which would be applied to wafers), three-enzyme polymers were prepared and stored in buffer at 37 °C. The enzyme polymers applied to the sensors contained up to 20 times more enzyme than used in the enzyme stability studies. Enzyme-containing polymers were prepared with 1000 units/g prepolymer of sarcosine oxidase and creatine amidinohydrolase and 2500 units/g prepolymer of creatinine amidohydrolase. The enzyme-polymers were removed periodically and assayed for oxygen consumption using an oxygen monitor when the gels were added to 1 mM of creatinine (Figure 23). Biopolymers lost only 50% of their activity after 11 days in buffer at 37 °C. Clearly, the three-enzyme polymers are very effective in utilizing creatinine and consuming oxygen.

When samples of enzyme gels were added to vials containing the amperometric sensor wafers, a faster inactivation occurred (Figure 23). This inactivation is likely to be caused by the presence of silver ions on the wafers. In fact, in similar experiments with sensor chips that were printed without AgCl, incubation caused no increase of inactivation. Based on the data showing the sensitivity of the three enzymes to silver ions and the soak test showing the inactivation of enzyme-polymers by silver chloride electrodes it is obviously important to prevent free silver from being in solution.

### Amperometric Creatinine Sensors and Stabilization to Silver

In order to test the three-enzyme biosensors using the enzyme-polyurethane membranes, amperometric creatinine sensors were prepared by applying enzyme-polymer directly to electrodes of wafers. Sensors were placed into sensor housing and tested for stability using a stop-flow apparatus. When not being tested, sensors were stored at 37 °C (wet). It was found that enzyme-polymers applied directly to the sensor-wafers quickly lost activity (Figure 24), whereas, enzyme-polymers stored in solution retained activity (as seen in Figure 23). This activity loss occurred regardless of whether or not the sensor was tested or stored untasted (indicating that hydrogen peroxide formation was not the cause of deactivation).

Since it is apparent that silver leaching from the electrodes can be the cause of inactivation, electrodes were prepared with a cellulose acetate cover membrane over the electrodes. Sensors with the cellulose acetate membranes had considerably improved half-lives (Figure 24). Further design of better membrane or the design of silver-free reference electrodes can further improve the use-life of these sensors.

While the invention has been described in conjunction with specific embodiments thereof, it is evident that many alternatives, modifications, and variations will be apparent to those skilled in the art in light of the foregoing description.

## Claims

1. A method for preparing a multiple-use three enzyme biosensor for the amperometric determination of creatinine in biological liquids, said biosensor comprising a plurality of immobilized enzymes, said method comprising applying to said biosensor an enzyme-polymer composition comprising said plurality of immobilized enzymes, said enzymes being immobilized by means of covalent binding, said method further comprising an initial step of chemically modifying said enzymes by attaching one or more polyethylene glycol (PEG) chains per enzyme monomer, wherein said plurality of immobilized enzymes comprise at least two of creatinine amidohydrolase, creatine amidinohydrolase and sarcosine oxidase.

2. The method of claim 1, wherein said enzymes are immobilized into the enzyme-polymer composition simultaneously.

3. The method of claim 1, wherein said enzymes are applied to said sensor simultaneously.

4. The method of claim 1, wherein prior to said modification said sarcosine oxidase is contacted with an inhibitor in an amount effective to prevent inactivation during modification,

5. The method of claim 4, wherein said inhibitor is pyrrole-2-carboxylic acid or (methylthio)acetic acid.

6. The method of any preceding claim, wherein said polymer is selected from the group consisting of polyurethane, polyvinyl chloride, polyester, polycarbonate, vinyl acetate copolymer, nylon, poly (1,4-butleneterephthalate), cellulose propionate, ethylene/acrylic acid copolymer, polybutadiene, polyethylene, polypropylene, polyimide, acrylic film, polystyrene, and polyvinyl fluoride.

7. The method of claim 4, wherein said enzyme-polymer composition comprises polyurethane.

8. The method of any preceding claim, wherein the biosensor comprises at least one working electrode, at least one reference electrode and at least one counter electrode.

9. The method of claim 8, wherein the enzyme-polymer composition is applied to said working electrode, said reference electrode and said counter electrode.

10. The method of claim 8 or 9, wherein said reference electrode reference electrode is an Ag/ AgCl electrode.

11. The method of claim 10, wherein said reference electrode is covered with a material that limits diffusion of silver ions emanating from said reference electrode, thereby preventing contact between the silver ions and the enzymes.

12. A method for preparation of an enzyme-polymer composition comprising
(a) chemically modifying a plurality of enzymes comprising at least one of(I)creatinine amidohydrolase (2) creatine amidinohydrolase and (3) sarcosine oxidase by attaching one or more poly(ethylene glycol) (PEG) chains per enzyme monomer, wherein prior to said modification said sarcosine oxidase is contacted with an inhibitor in an amount effective to prevent inactivation during modification, and
(b) contacting a solution containing said plurality of modified enzymes with a polymer solution under conditions allowing for polymerization, wherein said enzymes become covalently immobilized, thereby forming an enzyme-polymer composition.

13. The method of claim 12, wherein said inhibitor is pyrrole-2-carboxylic acid or (methylthio)acetic acid.

14. The method of claim 12 or 13, wherein said polymer is selected from the group consisting of polyurethane, polyvinyl chloride, polyester, polycarbonate, vinyl acetate copolymer, nylon, poly (1,4-butleneterephthalate), cellulose propionate, ethylene/acrylic acid copolymer, polybutadiene, polyethylene, polypropylene, polyimide, acrylic film, polystyrene, and polyvinyl fluoride.

15. The method of claim 14, wherein said enzyme-polymer composition comprises polyurethane.

16. A multiple-use three enzyme biosensor for the amperometric determination of creatinine in biological liquids comprising an enzyme-polymer composition comprising the enzymes creatinine amidohydrolase, creatine aminohydrolase and sarcosine oxidase, wherein at least one of the said enzymes is attached to one or more polylethylene glycol) (PEG) chains and immobilized into the composition by said one or more PEG chains.

## Patentansprüche

1. Verfahren zur Herstellung eines mehrartig verwendbaren Biosensors mit drei Enzymen zur amperometrischen Bestimmung von Creatinin in biologischen Flüssigkeiten, wobei der Biosensor eine Vielzahl von immobilisierten Enzymen umfasst, wobei bei dem Verfahren eine Enzym-Polymer-Zusammensetzung aus der Vielzahl von immobilisierten Enzymen auf den Biosensor aufgebracht wird, wobei die Enzyme durch kovalente Bindung immobilisiert werden, wobei das Verfahren ferner einen ersten Schritt der chemischen Modifizierung der Enzyme durch Anheftung von einer oder mehreren Polyethylenglykol-(PEG-)Ketten pro Enzymmonomer umfasst, wobei die Vielzahl von immobilisierten Enzymen zumindest zwei von Creatininamidohydrolase, Creatinamidinohydrolase und Sarcosinoxidase umfasst.

2. Verfahren nach Anspruch 1, wobei die Enzyme gleichzeitig in der Enzym-Polymer-Zusammensetzung immobilisiert werden.

3. Verfahren nach Anspruch 1, wobei die Enzyme gleichzeitig auf den Sensor aufgebracht werden.

4. Verfahren nach Anspruch 1, wobei vor der Modifizierung die Sarcosinoxidase mit einem Hemmer in einer wirksamen Menge zur Verhinderung einer Inaktivierung während der Modifizierung in Berührung gebracht wird.

5. Verfahren nach Anspruch 4, wobei der Hemmer Pyrrol-2-carbonsäure oder (Methylthio)essigsäure ist.

6. Verfahren nach einem der vorhergehenden Ansprüche, wobei das Polymer aus der aus Polyurethan, Polyvinylchlorid, Polyester, Polycarbonat, Vinylacetat-Copolymer, Nylon, Poly-(1,4-butylenterephthalat), Cellulosepropionat, Ethylen/Acrylsäure-Copolymer, Polybutadien, Polyethylen, Polypropylen, Polyimid, Acrylfilm, Polystyrol und Polyvinylfluorid bestehenden Gruppe ausgewählt ist.

7. Verfahren nach Anspruch 4, wobei die Enzym-Polymer-Zusammensetzung Polyurethan umfasst.

8. Verfahren nach einem der vorhergehenden Ansprüche, wobei der Biosensor zumindest eine Arbeitselektrode, zumindest eine Referenzelektrode und zumindest eine Gegenelektrode umfasst.

9. Verfahren nach Anspruch 8, wobei die Enzym-Polymer-Zusammensetzung auf die Arbeitselektrode, die Referenzelektrode und die Gegenelektrode aufgebracht wird.

10. Verfahren nach Anspruch 8 oder 9, wobei die Referenzelektrode eine Ag/AgCl-Elektrode ist.

11. Verfahren nach Anspruch 10, wobei die Referenzelektrode mit einem Material beschichtet ist, das die Diffusion von Silberionen aus der Referenzelektrode begrenzt, wodurch Kontakt zwischen den Silberionen und den Enzymen verhindert wird.

12. Verfahren zur Herstellung einer Enzym-Polymer-Zusammensetzung, das (a) die chemische Modifizierung einer Vielzahl von Enzymen, die zumindest eines von (1) Creatininmamidohydrolase, (2) Creatinamidinohydrolase und (3) Sarcosinoxidase umfasst, durch Anheftung von einer oder mehreren Polyethylenglykol-(PEG-)Ketten pro Enzymmonomer, wobei vor der Modifizierung die Sarcosinoxidase mit einem Hemmer in einer wirksamen Menge zur Verhinderung einer Inaktivierung während der Modifizierung in Berührung gebracht wird, und
(b) das Inberührungbringen einer Lösung, die die Vielzahl von modifizierten Enzymen enthält, mit einer Polymerlösung unter Bedingungen, unter denen Polymerisation möglich ist, umfasst, wobei die Enzyme kovalent immobilisiert werden und so eine Enzym-Polymer-Zusammensetzung bilden.

13. Verfahren nach Anspruch 12, wobei der Hemmer Pyrrol-2-carbonsäure oder (Methylthio)essigsäure ist.

14. Verfahren nach Anspruch 12 oder 13, wobei das Polymer aus der aus Polyurethan, Polyvinylchlorid, Polyester, Polycarbonat, Vinylacetat-Copolymer, Nylon, Poly-(1,4-butylenterephthalat), Cellulosepropionat, Ethylen/AcrylsäureCopolymer, Polybutadien, Polyethylen, Polypropylen, Polyimid, Acrylfilm, Polystyrol und Polyvinylfluorid bestehenden Gruppe ausgewählt ist.

15. Verfahren nach Anspruch 14, wobei die Enzym-Polymer-Zusammensetzung Polyurethan umfasst.

16. Mehrartig verwendbarer Biosensor mit drei Enzymen zur amperometrischen Bestimmung von Creatinin in biologischen Flüssigkeiten, der eine Enzym-Polymer-Zusammensetzung umfasst, die die Enzyme Creatininamidohydrolase, Creatinaminohydrolase und Sarcosinoxidase umfasst, wobei zumindest eines der Enzyme an eine oder mehrere Polyethylenglykol-(PEG-)Ketten angeheftet ist und durch die eine oder mehrere PEG-Ketten in der Zusammensetzung immobilisiert ist.

## Revendications

1. Procédé de préparation d'un biocapteur à trois enzymes à usage multiple, pour la détermination ampérométrique de créatinine dans des liquides biologiques, le biocapteur comprenant une pluralité d'enzymes immobilisés, le procédé comprenant l'application au biocapteur d'une composition d'enzyme-polymère comprenant la pluralité d'enzymes immobilisés, les enzymes étant immobilisés au moyen d'une liaison covalente, le procédé comprenant en outre un stade initial de modification chimique des enzymes en fixant une ou plusieurs chaînes de polyéthylène glycol ( PEG ) par monomère d'enzyme, la pluralité d'enzymes immobilisés comprenant au moins deux de créatinine amidohydrolase, créatinine amidinohydrolase et sarcosine oxydase.

2. Procédé suivant la revendication 1, dans lequel on immobilise les enzymes dans la composition de polymère d'enzyme simultanément.

3. Procédé suivant la revendication 1, dans lequel on applique les enzymes au capteur simultanément.

4. Procédé suivant la revendication 1, dans lequel avant la modification on met la sarcosine oxydase en contact avec un inhibiteur en une quantité efficace pour empêcher une inactivation pendant une modification.

5. Procédé suivant la revendication 4, dans lequel l'inhibiteur est l'acide pyrrole-2-carboxylique ou l'acide ( méthylthio )acétique.

6. Procédé suivant l'une quelconques des revendications précédentes, dans lequel le polymère est choisi dans le groupe consistant en un polyuréthane, en un poly( chlorure de vinyle ) en un polyester, en un polycarbonate, en un copolymère d'acétate de vinyle, en un nylon, en un poly( téréphthalate de 1,4 butylène ), en du propionate de cellulose, en un copolymère d'éthylène et d'acide acrylique, en un polybutadiène, en un polyéthylène, en un polypropylène, en un polyimide, en un film acrylique, en un polystyrène, et en un poly( fluorure de vinyle) .

7. Procédé suivant la revendication 4, dans lequel la composition d'enzyme-polymère comprend du polyuréthane.

8. Procédé suivant l'une quelconque des revendications précédentes, dans lequel le biocapteur comprend au moins une électrode de travail, au moins une électrode de référence et au moins une contre électrode.

9. Procédé suivant la revendication 8, dans lequel on applique la composition d'enzyme-polymère à l'électrode de travail, à électrode de référence et à la contre électrode.

10. Procédé suivant la revendication 8 ou 9, dans lequel l'électrode de référence est une électrode Ag/AgCl.

11. Procédé suivant la revendication 10, dans lequel on recouvre l'électrode de référence d'une matière qui limite la diffusion d'ions argent provenant de l'électrode de référence, en empêchant ainsi un contact entre les ions argent et les enzymes.

12. Procédé de préparation d'une composition d'enzyme-polymère, dans lequel :
( a ) on modifie chimiquement une pluralité d'enzymes comprenant au moins l'une de ( 1 ) créatine amidohydrolase, ( 2 ) de créatine amidohydrolase et ( 3 ) de sarcosine oxydase, en fixant une ou plusieurs chaînes de poly( éthylène glycol ) ( PEG ) par monomère d'enzyme, dans lequel avant la modification on met la sarcosine oxydase en contact avec un inhibiteur en une quantité efficace pour empêcher une inactivation pendant une modification, et
( b ) on met une solution contenant la pluralité d'enzymes modifiés en contact avec une solution de polymère dans des conditions permettant une polymérisation, les enzymes devenant immobilisés par covalence en formant ainsi une composition enzyme-polymère.

13. Procédé suivant la revendication 12, dans lequel l'inhibiteur est l'acide pyrrole-2-carboxylique ou l'acide ( méthylthio )acétique.

14. Procédé suivant la revendication 12 ou 13, dans lequel le polymère choisi dans le groupe consistant en un polyuréthane, en un poly( chlorure de vinyle ), en un polyester, un polycarbonate, en un copolymère d'acétate de vinyle, en un nylon, en un poly( téréphthalate de 1,4 butylène ), en du propionate de cellulose, en un copolymère d'éthylène et d'acide acrylique, en un polybutadiène, en un polyéthylène, en un polypropylène, en un polyimide, en un film acrylique, en un polystyrène, et en un poly( fluorure de vinyle ).

15. Procédé suivant la revendication 14, dans lequel la composition d'enzyme-polymère comprend du polyuréthane.

16. Biocapteur à trois enzymes à usage multiple, pour la détermination ampérométrique de créatinine dans des liquides biologiques, comprenant une composition d'enzyme-polymère comprenant les enzymes créatinine amidohydrolase, créatine aminohydrolase et sarcosine oxydase, dans lequel au moins l'un des enzymes est fixé à une ou à plusieurs chaînes de poly( éthylène glycol ) ( PEG ) et immobilisé dans la composition par la ou les plusieurs chaînes de PEG.
